# EUROPEAN PATENT APPLICATION

(11) **EP 2 202 522 A1**
(43) Date of publication of application: **30.06.2010**
(21) Application number: 08172871.9
(22) Date of filing: 23.12.2008
(51) Int. Cl.: G01N 33/554, G01N 33/551, G01N 33/543, G01N 30/92, B01L 3/00

(54) **Methods for immobilizing microvesicles, means and methods for detecting them, and uses thereof**

(71) Applicant: Universiteit Leiden, 2311 EZ Leiden (NL)
(72) Inventor: The designation of the inventor has not yet been filed
(74) Representative: Clark, David Julian

(57) **Abstract**

The invention provides a method for immobilizing microvesicles to a coated surdace using a laminar flow. Further provided are a chip mould for the preparation of microfluidic chips suitable for use in the method and uses thereof. Further provided is a microfluidic chip suitable for use of the method. Further provided is a coated surface suitable for use of the method and uses thereof. Further provided are means and methods for detecting microvesicles and uses thereof for detecting or determining diseases related to microvesicles. Further is provided a kit suitable for use of the method, comprising of a microfluidic chip and a coated surface.

## Description

The invention relates methods for immobilizing microvesicles, means and methods for detecting them, and uses thereof, a microfluidic chip for use therein and a chip mould for the preparation of a microfluidic chip.

Microvesicles (MVs) or microparticles (MPs) are fragments of plasma membrane ranging from 10 nm to 800nm shed from almost all cell types during activation and/or apoptosis. They are thought to originate directly from the plasma membrane of the cell or from cellular material and comprise the antigenic content of the cells which they originate from. It is shown *in vitro* that microvesicles are released from cells upon activation and apoptosis (1-5). Blood microvesicles are a population of microvesicles and may originate from different cell types and from different cellular compartment origins. As a consequence, different types of microvesicles are discerned. Different types of microvesicles comprise apoptotic bodies, exosomes and ectosomes. These different types of microvesicles originate from different cell types and also differ in content of lipids and proteins (Boulanger et al. Hypertension 2006; 48: 180-86.). The majority of blood microvesicles consists of platelet-derived microvesicles, characterized by their surface antigens, such as CD41 and CD61 (6, 7). The precise mechanism by which microvesicles are released has not yet been elucidated; however an increase in cytosolic calcium concentration may be necessary to trigger vesicle release (8). Some specific enzymes (floppase, scramblase) are thought to help in remodelling the plasma membrane by inducing the translocation of anionic phospholipids, such as phosphatidylserine (PS) from the inner membrane to the outer membrane (9;10). This event is followed by microvesicle release and cytoskeleton degradation by Ca2+-dependent proteolysis (11). Several studies point to a biological role of microvesicles as an inducer of angiogenesis and cancer metastasis (12-13). Microvesicles bearing active FasL have also been reported to induce lymphocyte apoptosis (14, 15). Our group and others have addressed the pro-coagulant features of microvesicles (7, 16-18).

In recent years blood microvesicles have increasingly received attention as a potential biomarker in the diagnosis and prognosis of disease. However, such studies have been hampered by the lack of accurate methods for the detection and quantification of microvesicles. Different methods and combinations of methods have been used including enzyme-linked immunoassays (ELISA) capturing microvesicles with immobilized annexin V or cell-specific antibodies (19-24) and flow cytometry (4, 5, 7, 16, 25). A drawback of ELISA methods is that it does not provide information on the actual numbers of microvesicles (27). Other disadvantages of ELISA methods are interference of soluble antigens and lack of information on size distribution. A disadvantage of flow cytometry (FACS) is that the size of microvesicles challenges the lowest detection limits of FACS and FACS is therefore inaccurate for determining a number of parameters of microvesicles (size, roughness etc.). The reason for the limited accuracy is that the laser light employed in the conventional flow cytometers which excites at a wavelength of 488 nm makes the measurement of the microvesicles smaller than 488 in diameter not reliable (28). There are at least two reasons why the detection of small microvesicles is not reliable employing methods using light scattering, such as FACS. In the first place, when using light scattering, the angular depedence of the scattering vanishes for small particles: small particles scatter light equally in all directions. Therefore, the intensity is only indirectly related to the size. Secondly, the number of fluorophores that can be bound to a small particle is less as the available surface is less. In both methods smaller particles contribute less to the total signal: the fluorescence signal is proportional to r² and the light scattering is proportional to r⁶ for small particles. Therefore, a FACS is not ideal for detecting microvesicles smaller than 488 nm (29). Other detection methods require the immobilisation of microvesicles on a flat surface to enable further detection steps. One of the problems is that immobilisation of a sufficient number of microvesicles on a surface for further detection purposes is difficult. High speed centrifugation is often used to achieve a sufficient yield, but a major drawback of high speed centrifugation is that it induces fusion of microvesicles (Langmuir 2007, 23, 9646-9550). Fusion affects number counts and it prevents establishing the origin of membrane bound proteins present in a microvesicle.

Surprisingly, the inventors have established a method which solves the above-mentioned problems. This invention provides a method to immobilize microvesicles ranging from 10-800 nm in diameter comprising steps of: coating a surface with molecules having an affinity for said microvesicles to obtain a coated surface; allow a fluid comprising said microvesicles to contact said coated surface by applying a laminar flow to said fluid to immobilize said microvesicles to said coated surface. An advantage of this method is that the number of microvesicles that is immobilized on the surface is higher than when using existing methods and therefore results in preparations with a higher density of microvesicles. Another advantage is that this method does not induce fusion of microvesicles. Another advantage is that a larger variety of different microvesicles is obtained using this method. Another advantage is that larger microvesicles are obtained when compared to other techniques. Another advantage is that the capture process is more efficient. Another advantage is that the method according to the invention allows for multiple passes over said coated surface. In a preferred embodiment, said method comprises a preprocessing step eliminating interfering material, preferably cells and/or proteins. An advantage thereof is the enhancement of the detection sensitivity and/or selectivity. In another embodiment, said method comprises fluid subfractionation. Preferably, subfractionating is performed using a microfluidic device. Another advantage is that also smaller microvesicles are obtained when compared to other techniques. Another advantage is that the method results in preparations containing less aspecific signals. The term microvesicles refers to particles having at least a bilipid layer and proteins. The term radius (r) and diameter refer to the radius/diameter determined with respect to the smallest length of a straight line through the center of a microvesicle from side to side of a microvesicle. Preferably, microvesicles are natural particles of a cellular origin. Microvesicles may originate from any cell type, i.e. animal cells, plant cells, yeast and bacteria. Any fluid comprising said microvesicles can be used as a source of microvesicles. Preferred sources are bodyfluids, such as blood, plasma, serum, cell suspensions, urine, synovial fluids, saliva, sputum or milk. An advantage thereof is that microvesicles can easily be isolated from fluids. Preferably, blood plasma or a product thereof is used as a source of microvesicles, because the presence of microvesicles or the number of microvesicles in blood plasma are indicative of the health status of an animal. In a preferred embodiment, platelet poor plasma is used as a source of microvesicles. Preferably, plateled poor plasma comprises fewer platelets than normal plasma. More preferably, platelet poor plasma comprises fewer than 10 * 10⁹ platelets per liter. An advantage thereof is that preparations containing few platelets are obtained, as presence of platelets complicates analysis. An example of a method for isolation of microvesicles from blood plasma is described in example 1. In another embodiment, preferably less than 2 ml of said fluid is used in the method according to the invention. An advantage thereof is that this limits the amount of sample material required. More preferably, said fluid has a volume of less than 100 microliter, preferably less than 10 microliter.

In a preferred embodiment said source of microvesicles is a source wherein the number of cells is depleted. In another preferred embodiment said source is a source, wherein the amount of proteins, preferably soluble proteins is depleted. An advantage of depleting cells and/or proteins is that analysis of microvesicles is facilitated, because cells and/or proteins complicate analysis. Methods of depleting proteins or cells in fluids are known in the art. In a preferred embodiment said fluid comprising microvesicles is concentrated by means of filtration prior to use.
In a preferred embodiment, microvesicles comprise exosomes. The term "exosome" refers to externally released vesicles originating from the endosomic compartment of cells. In preferred embodiments, said cells comprise tumor cells, endothelial cells or immune cells. Preferred is that said immune cells comprise antigen presenting cells, dendritic cells, macrophages, mast cells, T lymphocytes or B lymphocytes. Preferably, exosomes are of endosomal origin. Preferably, they are secreted in the extracellular milieu following fusion of late endosomal multivesicular bodies with the plasma membrane. Preferably exosomes have a size of 60-100 nm in diameter, or a density of 1,13 to 1,21 g/dL in a sucrose gradient. Preferred exosomes are characterized by an enrichment in HSP 70, tetraspanins, Tsg101, Alix and/or MHC molecules. An advantage of using exosomes is that (tumor-derived) exosomes are sometimes used as tumor antigen bearing vehicles. In addition, Toxoplasma gondii antigens pulsed DC-derived exosomes can be used for immunoprophylaxis. There is therefore a need in the art for improved methods of exosome detection.
In another preferred embodiment, microvesicles comprise ectosomes. The term "ectosome" refers to small right-side out vesicles released by cells by direct budding from the cell surface. Preferably, ectosomes are released by actived polymorphonuclear leukocytes (PMNs). Preferably, they bind complement. Preferred ectosomes are immune adherent similarly to large immune complexes or certain microorganisms. Preferably, ectosomes are characterized by their capability to down-modulate monocytes/macrophages activation in vitro similarly to apoptotic cells. Preferably, ectosomes are used as characterized according to Gasser et al. (Experimental Cell Research,Volume 285, Issue 2, 1 May 2003, Pages 243-257).
In another preferred embodiment, microvesicles comprise microvesicles which are released during apoptosis or other processes that compromise the integity of the outer cell membrane. In another embodiment, microvesicles comprise blood lipoprotein particles such particles are indicative of obesity and diabetes. Any molecule which has an affinity for microvesicles suitable for coating the selected surface material can be used. With a molecule having an affinity for microvesicles is meant that such molecule is capable of binding covalently or non-covalently to a molecule present on a microvesicle. Preferably said molecule present on a microvesicle is a membrane-bound molecule. Preferably, molecules having a high affinity for a microvesicle are used. Preferably, affinity is expressed as a dissociation constant. Preferably, molecules having a dissociation constant lower than 0.1 nM for microvesicles are used, more preferably lower than 10 nM. More preferably, molecules having a dissociation constant lower than 10 ^{- 15} M for microvesicles are used. In another preferred embodiment, an affinity for a microvesicle is used with a dissociation constant in a range between 0.1-10 nM. Methods of determining affinity are known in the art. Preferably, a method is used as described in Johnson et al. Journal of Molecular Biology 368 (2): 434-449.
Any surface that is suitable for immobilization using coatings having an affinity for microvesicles can be used Preferred surfaces are made of material comprising glass, mica, plastic, metal or ceramic materials. There are various methods known for coating surfaces having affinity for (glyco)-proteins, cell membranes or biomolecules in general. Typically, these methods use a reactive group which binds covalently or non-covalently to a certain biomolecule. For example, slides coated with aminoproplylsilane are used for non-covalent adsorption of protein. Epoxysilane coated slides are reactive with lysine, arginine, cysteine and hydroxyls at pH 5-9. Aldehyde coated slides are reactive with lysine and arginine where pH 7-10 drives Schiff's base reaction. A skilled person will know how to select the right coating suitable for use in combination with the selected surface material and test the affinity for microvesicles. The resulting coated surface is put into contact with microvesicles by applying a laminar flow to a fluid comprising said microvesicles. Said fluid can be any fluid that is compatible with microvesicles. With compatible is meant that the integrity of the microvesicles remains intact, which means that at least phospholipids and/or membrane proteins of the microvesicles are present. Preferably said fluid comprises plasma, cell culture medium, phosphate buffered saline (PBS), phosphate buffered potassium, or 4-(2-hydroxyethyl)-1-piperazineethanesulfonic acid (HEPES).

A laminar flow is a flow regime characterized by high momentum diffusion, low momentum convection, pressure and velocity independent from time. Said laminar flow is characterized by a Reynolds number of less than 2000 and higher than 0. Preferably, laminar flows with a Reynolds value between 0 and 1000, more preferably between 0 and 500 and most preferably between 0 and 100. A skilled person will know how to achieve a laminar flow. Any method capable of applying a fluid in a laminar flow to said coated surface can be used. Preferred is a laminar flow which is linear in one direction. Preferred is a laminar flow that optimizes the contact area, contact time and flow speed to the functionality of the fluidic device. Further preferred is a laminar flow through a channel comprising a functionalized wall.

Preferably, a method is used wherein the affinity for the microvesicles is specific. An advantage of a specific affinity is that binding of undesired molecules or particles is reduced. Preferred methods are methods in which an affinity linker is bound covalently or non-covalently to the surface. Binding an affinity linker to a surface may require a chemical treatment of said surface, depending on the material of said surface. Examples for different treatments of mica are provided in example 2 and 3. An affinity linker is a molecule that is capable to covalently or non-covalently bind to a binding partner, resulting in a complex between said affinity linker and said binding partner. Said binding partner can be a molecule capable of binding to a microvesicle or it can be a molecule present on the microvesicle that can directly interact with said affinity linker. Examples of affinity linkers and their binding partners are:
Streptavidin or avidin and biotin; an antibody and antigen; a ligand and receptor, lectin and saccharide, protein A and/or protein G-immunoglobulin constant region, and Tag peptide sequence and Tag antibody. The terms affinity linker and binding partner refer to their function. Therefore, depending on how the above mentioned affinity linker-binding partner combinations are used, the terms are exchangeable. For example a biotin can be an affinity linker if it is bound to the surface or it can be a binding partner when it is bound to the microvesicle. Any method to bind an affinity linker to a surface can be used. Methods to bind an affinity linker to a coated surface differ depending on the material of surface and the nature of the affinity linker. A skilled person will be able to select the correct method suitable for the type of surface material and affinity linker of choice. Methods to bind an affinity linker to a surface are known to a skilled person. Methods to bind antibodies to metal or silicon surface are well known in the art. Preferred methods are described in Bioelectrochemistry Volume 66, Issues 1-2, April 2005, Pages 111-115. Methods to bind antibodies to glass surface are also known and described in J Colloid Interface Sci. 2002 Aug 1;252(1):50-6. Preferably, the affinity linker is an antibody. Preferred methods of binding an affinity linker to a surface are provided in example 2 and 3. Preferred methods to attach antibodies to glass, silica or quartz are described in Pierce Tech Tip #5, 1/2006 on www.piercenet.com (downloaded 16 december 2008). A preferred method of binding an antibody to a surface comprises coating a surface with a DNA using methods for DNA immobilisation as known in the art, followed by allowing a fluid comprising said antibody fused with a nucleotide fragment having a complementary nucleotide sequence capable of hybridizing to said DNA immobilized to said surface to hybridize with said DNA. An advantage of said method is that it is significantly cheaper and faster

Preferably said surface is atomically flat. This is an advantage, because surface roughness is difficult to distinguish from signals caused by height of a microvesicle when using an Atomic Force Microscopy. For applications other than AFM and preferably for optical applications it is preferred that said surface is a flat surface, preferably an optically flat surface.

In a more preferred embodiment, said laminar flow is applied using microfluidics. The term microfluidics refers to devices, systems, and methods for the manipulation of fluid flows with characteristic length scales in the micrometer range up to 1 millimeter (see for a more complete overview: Manz, A. and Becker. H. (Eds.), Microsystem Technology in Chemistry and Life Sciences, Springer-Verlag Berlin Heidelberg New York, ISBN 3-540-65555-7). An advantage is that microfluidic systems possess the capability to execute operations more quickly than conventional, macroscopic systems, while consuming much smaller amounts of chemicals and fluids. A preferred method for applying a laminar flow using a microfluidic chip is provided in example 4.

In a more preferred embodiment, said laminar flow is created using a microfluidic chip. Preferably said microfluidic chip comprises at least one microfluidic channel with an inlet and an outlet, wherein said at least one microfluidic channel has at least one gap at a surface of said microfluidic chip enabling contact between said at least one channel and a surface. An advantage of said microfluidic chip is that it enables direct contact between a fluid in said microfluidic channel and a surface. Preferably said inlet and/or said outlet are positioned at a different surface of said microfluidic chip than the surface comprising said gap in said microfluidic channel. An advantage thereof is that this enables clamping between said microfluidic chip and said surface, while maintaining access to said inlet and/or outlet. In a preferred embodiment, holes are provided in said microfluidic chip, enabling screws or other means to attach a surface to said microfluidic chip. An advantage thereof is that a surface can be attached to said microfluidic chip to achieve a contact which prevents leakage of a fluid from said microfluidic channel. An advantage of using a microfluidic channel is that the geometry of the channel enables a controllable inducement of a laminar flow. Preferably, said channel has a height which is less than 1 mm. An advantage thereof is that the surface to volume ratio of said fluid over said coated surface is optimized. In another preferred embodiment, the channel height above the coated area is less than the channel height in other parts of the microfluidic device. An advantage thereof is that the surface to volume ratio of said fluid over the coated area is optimized, while maintaining a flow through. More preferably, said channel height is less than 0.5 mm. An advantage thereof is that this height is optimal for fluids having a viscosity of plasma. Preferably, said channel has a width of less than 1 mm. An advantage thereof is that this results in a minimal contact area of said coated surface which is in contact said fluid. Preferably, said minimal contact area is smaller than said coated surface area.
Preferably, said minimal contact area is between 100 and 10000 square micrometer. In a preferred embodiment, said minimal contact area is less than 1 square millimeter. An advantage thereof is that this limits the surface area inspection time and increases the concentration of collected vesicles per surface area. More preferably, said minimal contact area is between 1 square micrometer and 0.1 square millimeter.

In another preferred embodiment, said channel comprises a filter which allows microvesicles to pass through. A filter is defined as a structure having at least an opening allowing a particle with a size smaller than said opening to pass through, whereas larger particles cannot pass said opening. More preferably, said filter comprises micro pillars. A micropillar is a structure comprising substantially perpendicular projections, such as the micropillars disclosed in WO 03/103835. Said projections or micorpillars are preferably made of a non-porous substrate, and form projections substantially perpendicular to said channel, said projections having a height (H), diameter (D) and a distance or distances between the projections such, that a microvesicle can pass through between said projections. In addition to optimizing the above-mentioned height, diameter and a distance or distances between the projections, the projections may be given a desired chemical, biological or physical functionality, e.g. by modifying the surface of said projections.

In another preferred embodiment is a method wherein said channel comprises a filter which allows particles smaller than a microvesicle to pass through. In this method, microvesicles are collected on the surface or said filter, a change of flow direction is then applied to re-suspend said microvesicles. This method further comprises a step of resuspending said microvesicles in a fluid before allowing said fluid comprising said microvesicles to contact said coated surface.

In another preferred embodiment, said microfluidic chip comprises a pumping system. Any system suitable of pumping fluid inside a microfluidic circuit can be used. Examples are described in PHYSICS AND APPLICATIONS OF MICROFLUIDICS IN BIOLOGY David J. Beebe, Glennys A. Mensing, Glenn M. Walker Annual Review of Biomedical Engineering, August 2002, Vol. 4, Pages 261-286. In a preferred embodiment, said microfluidic chip comprises a deformable material to prevent leakage by means of self sealing. Such materials comprise, but are not limited to elastomers, preferably PDMS. In a preferred embodiment, said microfluidic chip comprises more than one channel. An advantage of a microfluidic chip comprising more than one channel is that different fluids can easily be applied to a (coated or uncoated) surface. In a preferred embodiment, said microfluidic chip has a dead volume of less than 10 microliter, more preferably less than 1 microliter. Dead volume is defined as the volume of the inner circuit which is not in direct surface connection with said coated surface and any of the volume of the inner circuit which is passed by said fluid after contacting said coated surface. Preferably, the volume of the residing capillary is excluded. In another embodiment the dead volume is defined as the volume in the inlet excluding the volume of the residing injected capillary.

In a more preferred embodiment said surface comprises mica. An advantage of mica is that it creates a molecular flat surface and can easily be cleaved.

In a more preferred embodiment, said molecules having an affinity for said microvesicles comprise antibodies or Annexin-V. An advantage thereof is that this results in more specific binding of microvesicles. Any antibody specific for microvesicles can be used. Preferably an antibody that is specific for an antigen on microvesicles is used. Preferably, said antibodies comprise antibodies which bind to membrane bound antigens present on cells or cellular parts thereof associated with the release of microvesicles including but not limited to haematopoietic cells, endothelial cells and/or malignant cells, More preferred antibodies comprise antibodies capable of binding to membrane bound antigens present on and/or antigens present in haematopoietic cells, preferably platelets, leukocytes, monocytes and/or erythrocytes. Other more preferred antibodies comprise antibodies binding to membrane bound antigens present on platelets, preferably anti-CD41, anti-CD41a, anti-CD42a, anti-CD42b, anti-CD40, anti-CD40L, anti-CD62P, anti-CD61 and/or anti-CD63. Other more preferred antibodies comprise antibodies capable of binding to membrane bound antigens present on or antigens present in leukocytes or cellular parts thereof, more preferably anti-CD3, anti-CD4, anti-CD8, anti-CD14, anti-CD11a, anti-CD11b, anti-CD20, anti-CD45, anti-CD66b, anti-CD66e, anti-CD68 and/or anti-CD162. Other more preferred antibodies comprise antibodies capable of binding membrane bound antigens present on or antigens present in erythrocytes or cellular parts thereof, more preferably , anti-CD235a. Other more preferred antibodies comprise antibodies capable of binding membrane bound antigens present on or antigens present in endothelial cells of cellular parts thereof, more preferably anti-CD31, anti-CD51, anti-CD54, anti-CD62e, anti-CD106, anti-CD144, anti-CD146, anti-CD105 or anti-vWF. Other more preferred antibodies comprise antibodies capable of binding membrane bound antigens present on or antigens present in malignant cells or cellular parts thereof, more preferably anti-MUC1, anti-CD133, anti-Fas, anti-FasL, anti-EGFR. Other preferred antibodies comprise antibodies capable of binding G- coupled proteins. Other more preferred antibodies comprise antibodies capable of binding membrane bound proteins present on or proteins present in MV and which are involved in a coagulation cascade. More preferred antibodies comprise antibodies capable of binding tissue factor (preferably membrane bound tissue factor), more preferably anti-CD142. Other more preferred antibodies comprise antibodies capable of binding to proteins or factors involved in cell signalling. Other preferred antibodies comprise antibodies capable of binding to proteins present on cellular parts of cells. Other more preferred are antibodies capable of binding antigens present on exosomes, preferably anti-CD63 and/or anti-CD81. In another preferred embodiment, said antibody comprises an anti-VE Cadherin or an anti-glycophorin A. Other preferred antibodies comprise antibodies capable of binding to antigens susceptible to viral particles (HIV-1), more preferably said antibodies comprise anti-CXCR4 and/or anti-CCR5. Other more preferred antibodies are capable of binding to antigens derived from a bacteria, a parasite, and/or a cellular prion protein (PrP(c)).

An advantage of these antibodies is that they capture certain subtypes of microvesicles and therefore their use results in low aspecific binding.
In another preferred embodiment, said surface is coated with an antibody directed against a constant domain of an antibody. An advantage thereof is that coated surfaces are obtained which can easily be coated with any antibody having an affinity for microvesicles. Preferred antibody directed against a constant domain of an antibody are antibodies directed against constant domains of IgG.

In a more preferred embodiment said method further comprises the detection of microvesicles using imaging. An advantage of this is that this allows measurements of one or more parameters comprising but not limited to particle geometry, shape, roughness, light scattering or dimensions of microvesicles or the presence of molecules on the surface of or inside microvesicles can be determined. Any method of imaging can be used in the method. Preferred methods of imaging comprise fluorescence microscopy, electron microscopy (EM), confocal microscopy, Raman spectroscopy, infrared spectroscopy or atomic force microscopy (AFM), or combinations thereof.

More preferably, said method comprises the detection using AFM. An advantage of AFM is that it is more accurate in detecting the size of microvesicles.

In another preferred embodiment of the invention, the detection is performed using an optical method. Any optical method may be used. Preferred optical methods comprise confocal microscopy, internal reflection fluorescence microscopy, ellipsometry/reflectometry, light scattering or surface plasmon microscopy. An advantage of said methods is that it can be implemented in high throughput detection methods. Even more preferably, these optical methods are calibrated using AFM measurements. An advantage thereof is that more reliable results are obtained.

Preferably, said calibration comprising determining the fluorescence light intensity relative to the microvesicle surface. Preferably, the absolute fluorescence light intensity of a surface comprising microvesicles with attached fluorescent antibodies is measured as well as the microvesicle surface concentration of said surface comprising microvesicles using AFM measurements. This calibration results in a specific fluorescence intensity per microvescicle. When using a method to produce said surface comprising microvesicles with attached fluorescent antibodies the concentration of microvescicles can be predicted by measuring said fluorescence intensity using said optical method.

Also preferred is detection combining use of AFM and optical methods or other imaging techniques. A preferred combination comprises the combination between AFM with LSCM. When AFM is combined with another type of optical detection method, measurements may be done separately for each detection method. A preferred combined use of said techniques has been described in Modern Research and Educational Topics in Microscopy by Adams and Czymmek Modern Research and Educational Topics in Microscopy, page 68-76, A Méndez-Vilas and J.Diaz (Eds.). Results of the measurements performed can be linked by storing the data together with position coordinates of the analysed microvesicles.

A preferred embodiment of the method further comprises a step wherein at least one species of antibodies is bound to the microvesicles. Said species of antibodies can be applied during the coating step before immobilization of microvesicles, in a step simultaneously together with the microvesicles or in a separate step after immobilization of said microvesicles. With a species of antibody is meant a detectable amount of antibodies directed against a single epitope, preferably said antibody is a monoclonal antibody. An advantage is that the presence of said species of antibodies on a microvesicle can be detected, which provides information about the presence of an epitope against which the antibody is directed on said microvesicle. Any species of an antibody directed against an epitope that is present on the microvesicle can be used. In another preferred embodiment, antibodies labeled with gold particles or other labels that can be detected with AFM or with fluorescence labels for fluorescence detection or any other label for these or any other detection methods are used. It is also possible to use antibodies labeled with multiple labels. An advantage thereof is that this enables detection of said antibody using different detection methods. In another preferred embodiment, said antibody is labeled with a molecule with an affinity linker for a special application of AFM, wherein the cantilever of the AFM is labeled with a binding partner of said affinity linker. An advantage thereof is that this application can be used to determine the specific interaction between the affinity linker and its binding partner. The adhesion force upon separation is then a measure of the binding strength. The detection of a positive adhesion force is therefore an alternative for detection techniques using labels that can be visualized by light or by size. An antibody can be used to capture microvesicles to a surface of the microfluidic chip and/or to label microvesicles that are bound to said surface.

In a more preferred embodiment, the range of the microvesicles is between 10 and 500 nm. An advantage of this range is that the detection accuracy is better in this range. This can be important when the size of microvesicles is analyzed for determining the presence of deviate microvesicles, for example in the plasma of individuals suffering from diseases associated with microvesicles. More preferably the range is 10-200 nm, because within this range the detection accuracy is even better. Best detection accuracy can be obtained when using microvesicles in a range between 60 and 80 nm. More preferably, microvesicles with a diameter typically around 65 nm are used. An advantage thereof is that the majority of microvesicles have a diameter around 65 nm. A more preferred embodiment is a method wherein a first section of said surface with the microvesicles immobilized thereto is exposed to a first species of antibodies, whereas the remaining section is not exposed. An advantage of this method is that a surface is obtained with a section wherein vesicles are labeled with antibodies and a section that is not labeled and thereby serving as a negative control.

More preferably, said method further comprises a second section which is exposed to a second species of antibodies. An advantage is that a further section is obtained which is labeled with a second species of an antibody. Therefore, a negative control a positive labeling for said first species, a positive labeling for said second species is obtained on a single surface. This enables easy comparison. A skilled person will understand that in the same manner also further species of antibodies can be used on a single surface.

In another preferred embodiment, said fluid is allowed to contact a first and a second section labeled with a first and a second species of antibodies. Preferably, the length of said first section and said second section is more than twice the width of said channel. The length of a section is defined as the length between the beginning of said first section or said second section which is contacted first by said fluid and the end of said first section or said second section, which is contacted the last by said fluid. An advantage thereof is that in this embodiment not all microvesicles are collected in said first section. Therefore, said second section can be used to determine the affinity of a subset of said vesicles in said fluid for said second species of antibodies.

In another preferred embodiment, said fluid is allowed to contact at least a first and a second section labeled with a first species of antibodies. An advantage thereof is that microvesicles in said fluid are contacted to said first section, therebye capturing a proportion of said microvesicles on said first section and further capturing another proportion of said microvesicles in said second section. By determining the difference in the number of microvesicles between said first and said second section captured, the concentration of microvesicles in said fluid can be determined.

The invention further provides a microfluidic chip, comprising at least one microfluidic channel with an inlet and an outlet, wherein said at least one microfluidic channel has at least one gap at a surface of said microfluidic chip enabling contact between said at least one channel and a surface. An advantage of said microfluidic chip is that it enables direct contact between a fluid in said microfluidic channel and a surface.

The invention further provides a method for constructing a mould for a microfluidic chip, said method comprising providing:
- a first microfluidic chip mould comprising a bottom, and surrounding sides to form a first receptacle for fluid,
- wherein at least one side, preferably the top is at least partly open to the exterior of the first mould to allow filling of the first receptacle of the first mould,
- said bottom containing an image of a channel, preferably a fluid inlet, a fluid outlet, and preferably one or more chambers, wherein said preferably a fluid inlet, fluid outlet and said preferably one or more chambers are in fluid connection with said channel,
- wherein said image is present on a surface of the bottom that is exposed to the fill side of said first receptacle,
- wherein said image comprises material that is in immediate contact with the surface of the bottom that is exposed to the fill side of said first receptacle,
   said method further comprising
- filling said first mould with a first microfluidic chip casting material,
- curing the first casting material to create a first cast comprising a first microfluidic chip,
- separating the thus formed first microfluidic chip from the first mould,
   providing the first microfluidic chip with surrounding sides to form a second receptacle for fluid,
- wherein at least one side of the second receptacle, preferably the top is at least partly open to the exterior to allow filling of the second receptacle,
- wherein the surface of the first microfluidic chip containing said channel, fluid inlet, fluid outlet and preferably one or more chambers is exposed to the fill side of the second receptacle,
- filling said second receptacle with a second microfluidic chip casting material,
- curing the second casting material thereby creating a second cast comprising a second mould for said microfluidic chip, and separating said second mould from said first microfluidic chip,
- wherein said second mould comprises a copy of the image of the first mould.

In a preferred embodiment the top of said image is essentially flat. An advantage thereof is that said top can be polished. Polished images result in atomically flat surfaces of a channel, which is important in achieving a laminar flow with low Reynold values.

Preferably, said first microfluidic chip mould is made of a metal, preferably brass, wherein the image of a channel is produced by lithographic means. This allows the generation of a channel suitable for the production of a microfluidic chip.

An image is the material inversion of the open space within the outer surfaces of said microfluidic chip. Preferably, an image is formed by all material protruding from the flat sides of the mould exposed to the fill side a receptacle. An image can be material protruding from all sides of said mould. Said first microfluidic chip casting material can be any material which can be polymerized and is compatible with microvesicles. Preferably, the first cast (the first microfluidic chip) and the third cast are made of deformable material. Preferably, said material comprises PDMS. Said second cast (the second mould) is made of a material which can be solidified. Preferably said material can be polymerized. In a more preferred embodiment said method further comprises adapting said second mould and/or by removing material from said second mould by adding material to said second mould. An advantage of a method according to the invention is that adapting a mould which can be polymerized is easier than adapting microfluidic chip moulds known in the art. Moulds made using lithographic methods typically produce height differences of typically 30 microns. Producing greater differences in height is very time consuming and not easy. Removing material from a mould made of a material which can be polymerized to create a structure requiring a height difference of more than 30 microns can easily be produced by adapting said second microfluidic chip mould. Another advantage is that drilling into a mould made of a material which can be polymerized is possible from all directions, without risking damage of the structure of said image.
Preferably the material of said second cast comprizes polyurethane. In another embodiment the material of said second cast comprizes a thermoplast. In another embodiment the material of said second cast comprizes ABS, PMMA (perspex), Telfon, polycarbonate and/or polysulfone. Preferably said first cast and said second cast are made of material that can be separated from each other after curing. Preferably said second and said third cast (the second microfluidic chip) are made of material that can be separated from each other after curing.

Preferred is a method, wherein said image comprises material that protrudes from said surface of the bottom that is exposed to the fill side of the first receptacle.

In a preferred embodiment said first cast and said second cast are made from polymers that can be separated from each other after curing. Preferably said first cast comprises PDMS. In a preferred embodiment said second cast comprises polyurethane.

A preferred embodiment is a method comprising providing said second mould with surrounding sides to form a third receptacle for fluid,
- wherein at least one side, preferably the top of said third receptacle is at least partly open to the exterior to allow filling of the third receptacle, and
- wherein the side of said second mould that comprises said image is exposed to the fill side of said third receptacle,

A preferred embodiment is a method further comprising filling said third receptacle with a third microfluidic chip casting material and curing the third casting material to create a third cast comprising the second microfluidic chip, wherein said second cast and said third cast are made from polymers that can be separated from each other after curing.

The invention further provides a mould for a microfluidic chip cast of a deformable material, obtainable by a method according to the invention. The mould preferably comprises sides surrounding said mould to form a receptacle for fluid, wherein at least one side, preferably the top of the receptacle is at least partly open to the exterior to allow filling of the receptacle. The side that is exposed to the fill side of the receptacle is the side that contains the image of the channel, fluid inlet, fluid outlet and preferably one or more chambers, wherein said fluid inlet, fluid outlet and said one or more chamber (if present) are in fluid connection with said channel.

Preferred is a mould for a microfluidic chip, wherein said deformable material is PDMS.

The invention provides the use of said chip mould for the preparation of a microfluidic chip. An advantage of using a chip mould is that it allows easy production of microfluidic chips.

The invention further provides a method for the preparation of a chip mould according to the invention, comprising steps of covering a microfluidic chip with a polymer, allow said polymer to polymerize and separate said polymerized polymer from said microfluidic chip. Any microfluidic chip suitable for a method of the invention can be used. Preferably, said microfluidic chip comprises a microfluidic chip comprising PDMS. Any polymer can be used which can be separated from said microfluidic chip after setting of said polymer. Preferably, said polymer is polyurethane.

In another aspect the invention provides a coated surface suitable for use of the method. Preferably, said coated surface is prepared using a microfluidic chip of the invention.

Preferably, said coated surface is used for determining a parameter of microvesicles in a biological sample of an animal, wherein the parameter comprises the number, the dimensions and/or the protein factors of said microvesicles. An advantage of this use is that a coated surface can easily be used for determining said parameters. Any biological sample can be used wherein microvesicles are present. Preferably, said biological sample is obtained from synovial fluid, whole blood, plasma, serum, lymph, spinal fluid and urine, because said samples can easily be obtained. A sample of any animal can be used. Preferably said animal is a human.

More preferably, said coated surface is used for determining a parameter for determining whether said individual is at risk of suffering or developing a disease associated with the presence of microvesicles. An advantage of this use is that it enables easy determination of a parameter for determining whether said individual is at risk of suffering or developing a disease associated with the presence of microvesicles. Another advantage of using said coated surface is that the number of microvesicles immobilized to said coated surface is proportional to the concentration of microvesicles in said fluid. Therefore, a person skilled in the art is able to calculate said concentration based on said number of microvesicles. Of course, a skilled person can also determine based on such parameter a degree of suffering of or recovering from a disease associated with the presence of microvesicles. Preferably, a disease associated with the presence of microvesicles comprises a disease wherein said parameter is different in said animal suffering from a disease than a healthy individual. Preferred diseases are characterized by the presence of an increased number of microvesicles, other diseases are characterized by the presence of specific types of microvesicles. For example, in Tesselaar et al, Journal of Thrombosis and Haemostasis 2007, is described that the presence of MUC1 bearing microvesicles is associated with a poor survival in patients with metastatic breast and pancreatic cancer patients.

More preferably, said disease comprises an inflammatory disease, more preferably a systemic inflammatory disease including an autoimmune disease, diabetes mellitus or immune-mediated thrombosis, diabetes, more preferablt diabetes mellitus, a kidney disease, a cardiovascular disease, more preferably an acute coronary syndrome, diabetes mellitus, a neoplasm, more preferably cancer or a malignancy, more preferably a solid tumor or a leukemia, an infectious disease, more preferably a viral infection, a bacterial infection or a parasitic disease, a haematological disease, more preferably a sickle cell disease, a respiratory disease, a nutritional or metabolic disorder, an endocrine disease, an immunological diseases, a neurodegenerative disease or a neurological disorder. More preferably, said disease comprises diseases with vascular involvement and hypercoagulability such as disseminated intravascular coagulation,

In another aspect the invention provides the use of a microfluidic chip for the immobilization of microvesicles to a coated surface according to the invention. An advantage of using a microfluidic chip is that it is a single device ready for use in the method and enables a high yield of immobilized microvesicles while using only a small volume of fluid. Said microfluidic chip can also be used to apply a coating to a surface or to apply antibodies to said microvesicles. A preferred use of said microfluidic chip is described in example 4.

In another aspect, the invention provides a kit suitable for use of the method, comprising of a microfluidic chip and a coated surface.

### Examples

### Example 1: Blood collection and platelet poor plasma isolation

Blood was collected from seven donors between 8.00-10.00 A.M. and processed within 10-15 minutes. Venipucture was performed using a 21 gauge needle (BD vacutainer, San Jose, CA) and minimal stasis. After discarding the first four ml of blood, blood was collected in 1/10 volume of sodium citrate (3.2%, 0.105 M) using a 4.5 ml vacutainer tube (Becton Dickinson, San Jose, CA). Blood was centrifuged at 2,000xg for 10 min at 20°C, without brake. Plasma was carefully collected and centrifuged again at 2,000xg for 10 min, 20°C, without brake, to obtain platelet poor plasma (PPP). PPP was aliquotted in 250 µl portions. For the isolation of microvesicles (MVs) from fresh PPP, PPP was directly subjected to the next centrifugation steps. Otherwise, PPP was snap frozen in liquid N₂ and stored at -80°C until used for MP isolation.

### Isolation of Microvesicles

For AFM measurement, 750 µl PPP was pooled and centrifuged at 18,890xg, 20°C for 30 minutes with minimum brake. The supernatant was removed carefully except for 25 µl containing the MV pellet. This pellet was resuspended with 1 ml Hepes buffer (10 mM Hepes (Merck, Germany), 137 mM NaCl (Merck, Germany), 4 mM KCl (Merck, Germany), 0.1 mM Pefabloc® SC (Fluka, Germany), pH 7.4), vortexed, and centrifuged as before. The supernatant was removed leaving a volume of 25 µl containing the MV pellet. This 25 µl was vortexed and then used for AFM measurement.
For FACS measurement, 250 µl PPP was centrifuged at 18,890xg, 20°C for 30 minutes with minimum brake and then the supernatant was carefully removed leaving a volume of 25 µl containing the pellet. To wash MVs, 225 µl of Hepes buffer was added and then this mixture was centrifuged again at 18,890xg, 20°C for 30 minutes with minimum brake. The supernatant (225 µl) was removed and the residual 25 µl was diluted to 100 µl with Hepes buffer. This MV preparation was directly used for staining with antibodies.

### Example 2: Modification of mica for immobilizing anti-CD41 antibody

The surface of mica was modified as follows: a solution was made containing 55% (w/v) ethanolamine (Sigma Aldrich, Germany) in DMSO (99.7% purity; Biosolve BV, Netherlands). Molecular sieve beads (0.3 nm; Sigma Aldrich, Germany) were added to adsorb the water formed later during the amine reaction on the mica surface. This solution was heated at 70°C until ethanolamine was completely dissolved. Next, freshly cleaved muscovite mica sheets (Electron Microscopy Sciences, Washington) having a diameter of 10 mm were incubated overnight in the ethanolamine solution.
Subsequently mica sheets were rinsed twice in DMSO (99.7% purity, 70°C) and twice in ethanol absolute (Biosolve BV, Netherlands) at room temperature (RT). These mica sheets were dried under N₂ flow. At this point, the amine modified mica can be used immediately for the next modification step or stored in a desiccator for up to several weeks. Prior to protein coupling, the modified mica was incubated three hours with 1 mg/ml Ethylene glycol-bis-(2-aminoethyl)-N,N,N', N'-tetraacetic acid (EGTA) (Sigma Aldrich, Germany) in chloroform (Merck, Gemany) containing 0.5% v/v triethylamine (Merck, Germany). Next, the mica sheets were washed in chloroform and dried under N₂ flow, then glued on a steel disc (diameter = 12 mm) with a silicon based glue (Rhodia silicon, Germany). Fifty µl of mouse anti-human CD41 antibody clone P2 (Beckman Coulter, Fullerton, CA) (0.01 mg/ml) was applied to the modified mica and incubated for 30 minutes at RT. Excess of anti-CD41 was removed by washing with Hepes buffer. As a negative control, mouse IgG1 pure clone X40 (Becton Dickinson, San Jose, CA) was used (0.01 mg/ml).

### Example 3: AFM analysis of CD41⁺microvesicles

Twenty µl of MV fraction was applied to anti-CD41-coated mica surface and incubated for 30 minutes to allow the binding of MVs to anti-CD41. To remove unbound MVs mica was washed twice with Hepes buffer. During the incubation step and before imaging, the mica surface was kept in a closed container.
Before use, the AFM glass fluid cell (Veeco, NY) was sonicated for 5 minutes in Sodium Dodecylsulfate (SDS) (Serva Electrophoresis, Germany), rinsed with distilled water, and allowed to dry under N₂ flow. A micro cantilever silicon tip (Olympus, Japan) with a spring constant of 2 N/m and 70 kHz resonant frequency was attached to the fluid cell. After mounting the steel disc containing the sample on the piezoelectric scanner of AFM, 80 µl of imaging buffer containing 20 mM Tris (Roche, Switzerland) and 150 mM KCl (pH 7.4) was added. The fluid cell with the cantilever attached was then seated against the sample. AFM (Veeco, NY) operated in fluid-tapping mode was performed to scan the topography of the mica and attached MVs thus acquiring three-dimensional images of the MVs. With an imaging rate of 1.5 Hz the surface was scanned at different positions to obtain 4 to 10 images with a scan size of 100 µm² per image. Images were captured and recorded using Nanoscope software version 5.30r2.
AFM data was analysed using SPMediator version 6.1 software (courtesy of Dr. S.J.T. van Noort) that evaluates the centre of mass of particles in comparison with the surface background. This software automatically calculates the volume and z dimensions (height) of MVs. From the MV volume, the MV diameter was calculated assuming a spherical shape.
To calculate the number of CD41⁺MVs/ L PPP detected by AFM, the following formula was used: (10⁶/750) x (25/20) x (78.5x10⁶/100) x N̅AFM, in which 750 (µl) is the original volume of PPP for MVs isolation, 25 (µl) is the volume of MV suspension, 20 (µl) is the volume of the MVs suspension added on the mica surface, 78.5x10⁶ (µm²) is the surface area of the mica, 100 (µm²) is the surface area of an AFM image, and NAFM is the mean of the total number of CD41⁺MVs per image after correction for the number of MVs found on IgG1 control-coated mica.

### FACS analysis of CD41⁺microvesicles

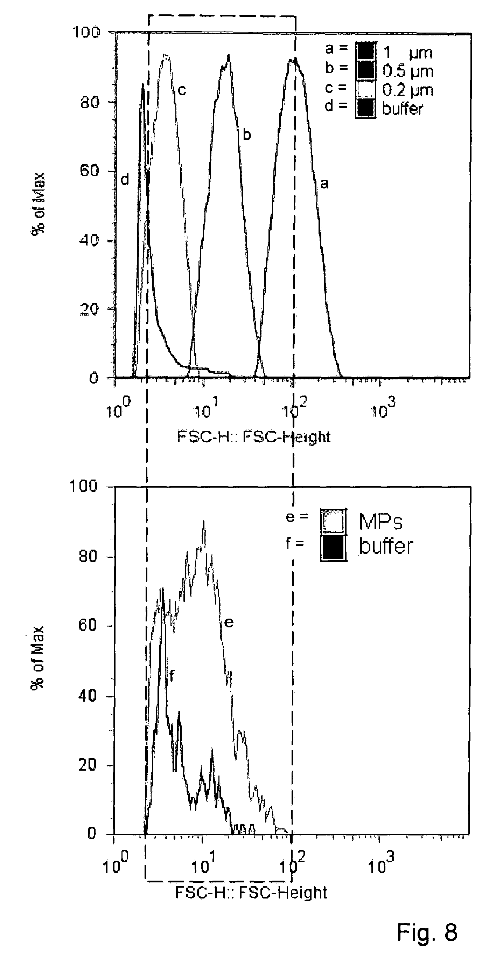

FACS analysis was performed using a FACS Calibur flow cytometer with CellQuest pro software (Becton Dickinson, San Jose, CA). To calibrate the flow cytometer and to set the scatter parameters for MVs analysis, size standard polystrene beads of 0.2, 0.5, and 1 µm (Fluka, Germany) were used. When MVs were measured, the forward size scatter of MVs showed that MVs with a size of 0.2 µm and above could be detected (Figure 8).
Five µl MV fraction was incubated with 5 µl phycoerythrin (PE) labelled mouse anti-human CD41 clone P2 (1:100 dilution) (Beckman coulter, Fullerton, CA) in 40 µl of Hepes buffer (containing 137 mM NaCl, 4 mM KCl, and 0.1 mM Pefabloc®) for 30 minutes at RT in the dark. Next, 200 µl Hepes buffer was added and the MV suspension was centrifuged at 18,890xg, 20°C for 30 minutes with minimum brake. The supernatant was removed carefully and the residual 50 µl was diluted to 350 µl with Hepes buffer. As negative control, mouse IgG1 PE clone X40 (Becton Dickinson, San Jose, CA) was used at the same concentration as the anti-CD41 antibody. For quantification of CD41⁺MVs per L, we used FACS absolute count standard beads with a mean diameter of 7.58 µm/bead and a known concentration (Cbeads) (Bang Laboratories, Fishers, IN) as an external standard. Two hundred µl of these beads (Vbeads) was mixed with Hepes buffer to a volume of 600 µl (Vtot) and then measured in triplicate (N̅beads). For measuring counting beads as well as MVs, the flow cytometer was programmed to count events in one minute. To calculate the volume (Z) measured by FACS, the following formula was used: Vtot/Vbeads x (Nbead̅s̅/Cbeads). The amount of CD41⁺MV/ L of PPP detected by FACS was calculated as: (350/Z) x (100/5) x 10⁶/250 x NFACS, i̅n̅ which 350 (µl) is the total volume of MVs before analysis, 100 (µl) is the total volume of the original MV suspension, 5 (µl) is the volume of MV used for antibody labelling, 250 (µl) is the original volume of PPP for MV isolation, and NFACS is the̅ mean number of CD41+ events counted by FACS, after correction for IgG1 control background events.

### Statistical analysis

Statistical evaluation was performed using SPSS 14.0. Correlation of the amount of MVs in PPP measured by AFM and FACS was tested with the Pearson and Spearman correlation tests.

### RESULTS

### AFM

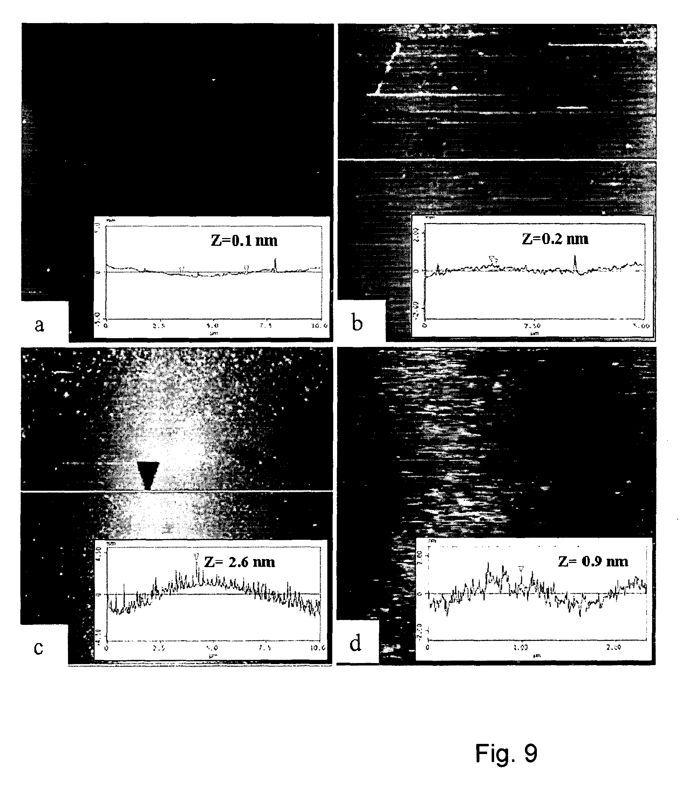

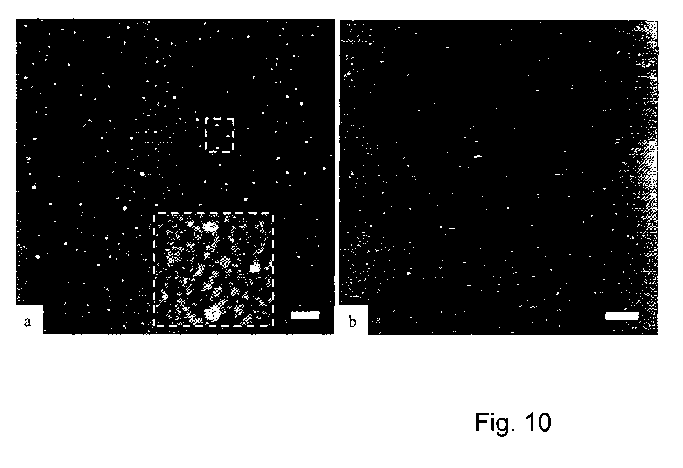

The surface of cleaved mica scanned with AFM operated in fluid-tapping mode was flat with an apparent height of ∼0.1 nm (Figure 9a). After modification of this surface, the surface topography was still nearly flat with a measured height of ∼0.2 nm (Figure 9b). Following the immobilization of mouse anti-human CD41 antibody to the modified mica, an apparent height of ∼2.6 nm of the anti-CD41 antibody was detected (Figure 9c). As a negative control, a mouse IgG1 isotype control was used at the same concentration as the mouse anti-human CD41 antibody. The surface topography scan showed an apparent height of ∼0.9 nm of the mouse IgG1 isotype control (Figure 9d).
Imaging with AFM demonstrated specific binding of MVs isolated from fresh PPP to anti-CD41-coated mica. IgG1 isotype control coated mica was used to control for non-specific MVs binding. Three dimensional AFM topography showed that there were spherical vesicles, defined as MVs, attached to the anti-CD41-coated mica while these were virtually absent on the IgG1 isotype control-coated mica (Figure 10a,b).
Variation of the incubation time of MVs with the anti-CD41-coated mica (2, 15, 30, and 45 min) showed that the total number and size distribution of specifically attached MVs had reached a plateau after 15 minutes (323 counts/100 µm²).

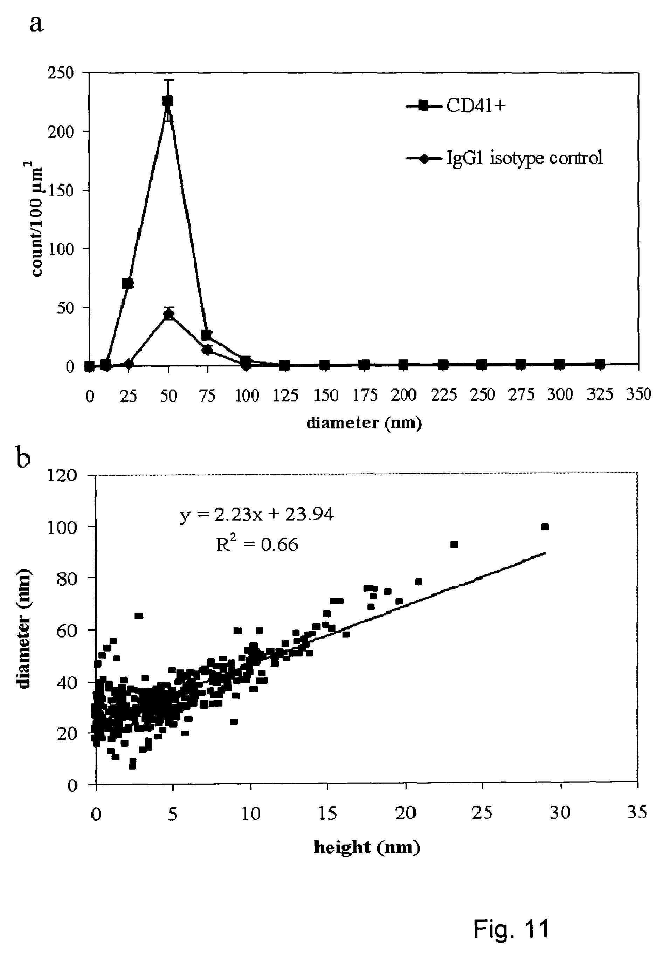

Figure 11 shows the results of a representative experiment. Figure 11a shows that the MVs attached to the anti-CD41-coated mica have diameters ranging from 10-325 nm. The diameter of the majority of these MVs was about 50 nm. In figure 11b the apparent height of CD41⁺MVs is plotted on the x axis as a function of their corresponding diameter on the y axis. The plot showed that the diameter size of the CD41⁺MVs exceeded the height approximately tenfold. Therefore to define the size distribution of MVs, the analysis of AFM data with the SPMediator version 6.1 software was based on the volume of MVs assuming a spherical shape.
To investigate the relationship between MV concentration and number of CD41⁺MVs detected by AFM, different dilutions (D) (D= 1, 2, and 4) of a MV preparation isolated from fresh PPP were incubated with anti-CD41-coated mica and imaged by AFM. A linear relationship (Y = 286.88 X + 34.91 counts/100 µm², R2 = 0.98) was obtained between the number of CD41⁺MVs (Y) detected per 100 µm² mica and the MV concentration (X= 100/D). From this linear relationship, it could be predicted that about 35 MVs attach non-specifically to 100 µm² of IgG1 isotype control-coated mica.
To determine the reproducibility of the measurement of CD41+MVs with AFM, MVs were isolated from frozen-thawed PPP on three consecutive days and analysed by AFM for the presence of CD41+ MVs. From this experiment, we estimate that the between assay variation for MVs isolation and AFM measurement (including mica modification) was 16% (mean 528x10⁶ CD41⁺MVs/L; SD 83x10⁶ CD41⁺MVs/L).
To investigate the effect of freezing and thawing on the number and size distribution of CD41⁺MVs measured by AFM, MVs were isolated from PPP of three donors before and after freezing-thawing. For two donors there was no significant difference in size distribution of the CD41⁺MVs measured by AFM before and after freezing-thawing, although the number CD41+MVs decreased (2- and 7-fold, respectively). For one donor we found a two-fold increase in number and a slight shift in size distribution of the CD41⁺MVs in MVs isolated after freezing and thawing of the PPP (from 50-250 nm to 10-200 nm).

### Flow cytometry

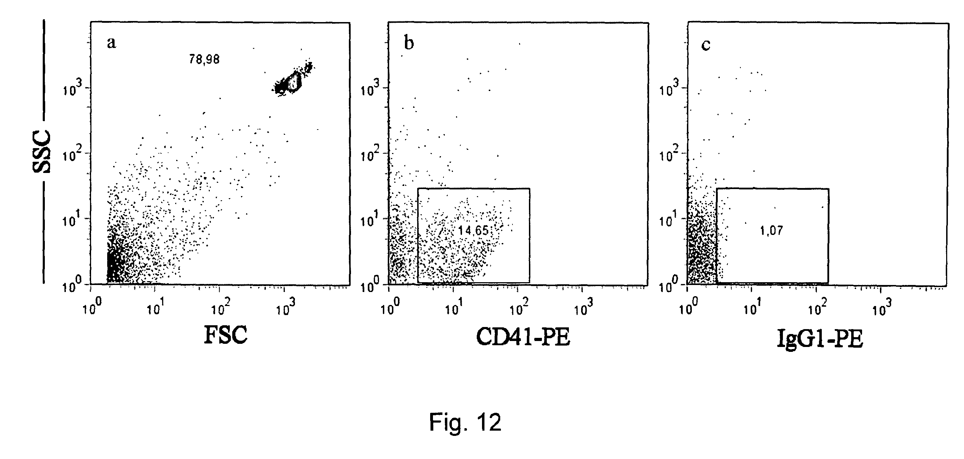

The CD41⁺MVs were quantified by FACS using a known concentration of counting beads as external standard in the measurement. The singlet population of these beads could be detected as shown in the forward scatter plot of Figure 12a. By counting the number of beads, the volume used for the measurement could be calculated. In all experiments the mean volume analyzed by FACS was 80±4 µl.
MVs were characterized by their CD41 expression (Figure 12b, c). The number of CD41⁺MVs was calculated after correction for the number of MVs staining with the IgG1 isotype (negative control). The reproducibility of MVs measurement with FACS was assessed on three consecutive days by one single operator performing the CD41⁺MVs measurement in triplicate. For these experiments, MVs were isolated from aliquots of frozen PPP. The variation in the number of CD41⁺MVs was 10% (mean 0.40x10⁶ MVs/L, SD 0.04x10⁶ MVs/L).
The effect of freezing and thawing on the number of CD41⁺MVs measured by FACS was also assessed. Flow cytometric analysis of MVs isolated from PPP of 4 donors before and after thawing revealed that depending on the donor 2-10 fold more CD41⁺MVs were detected in the MVs fraction prepared from frozen-thawed PPP than in the MVs isolated from fresh PPP.

### AFM versus flow cytometry

MVs were isolated from fresh PPP of 7 donors (MVs1-7). From one donor, PPP for MV isolation was obtained on three different days (MVs 1a, b, c). All 10 MV preparations were analysed simultaneously by AFM and FACS for numbers and size distribution of CD41⁺MVs.
The numbers of CD41⁺MVs obtained by AFM and FACS are shown in Table 1. Depending on the MV preparation 4-10 images of 100 µm² were scanned. About 255 CD41+MVs were detected by AFM per 100 µm² of the anti-CD41-coated mica surface whereas about 19% of these MVs (mean 48 counts/100 µm² of MVs) attached non-specifically to IgG1 isotype control-coated mica. We found that the number of CD41⁺MVs detected by AFM (32-702 x 10⁹/L PPP) was about 1000-fold higher than the number of CD41+MVs detected by FACS (11-291 x 10⁶/L PPP). In all seven donors the size of the CD41⁺MVs measured by AFM ranged from 10-475 nm. The majority of these MVs have a diameter of about 67.5±26.5 nm. On the IgG1 isotype control-coated mica particles were found ranging from sizes 10-200 nm, with the majority of MVs having a measured diameter of 53.8±13.4 nm.
The seven donors have a quite high variation in their number of CD41⁺MVs (75% for AFM measurements and 86% for FACS measurements). There was only a weak correlation between the number of CD41⁺MVs detected with AFM and FACS (r=0.17, P>0.05).

### DISCUSSION

In this example, we report a method for the detection of CD41⁺MVs with AFM. We have demonstrated that the number of CD41⁺MVs detected by AFM is about 1000-fold higher than the number detected by FACS. Our method of mica modification, and detection of CD41⁺MVs on anti-CD41-coated mica with AFM resulted in reproducible numbers of CD41⁺MVs (CV= 16%). The high interindividual variation in the number of CD41⁺MVs isolated from fresh PPP (75% for AFM and 86% for FACS) mainly reflects biological variation. Immobilization of mouse anti-human CD41 antibody and of mouse IgG1 isotype as a control on modified mica was successful, and a linear relationship between the MV concentration and the number of CD41⁺MVs as detected by AFM was demonstrated. Binding of MVs to anti-CD41-coated mica seemed specific since we only found much less MVs bound to IgG1 isotype control-coated mica in repeated experiments (see table 1). These non-specifically bound MVs were found to have a slightly smaller diameter range (53.8±13.4 nm). At this moment we do not know what these objects are, but they might be contaminants from the environment or non specifically bound (aggregates of) plasma proteins which still present in the MV preparation.
With AFM, we not only obtained the information of the numbers but also on the shape and size distribution of CD41⁺MVs. Using AFM we counted CD41+ particles with diameter sizes ranging from 10 to 475 nm, while with FACS these nanosized CD41⁺MVs are not detectable. This might, in part, explain why the numbers of CD41⁺MVs isolated from fresh PPP and detected by AFM were about 1000-fold higher than those detected by FACS. A similar observation was made by Zwicker (35) who reported that the number of tissue factor-bearing microvesicles observed by light scatter FACS was 10,000-fold less than what they observed using an impedance-based flow cytometer. Apparently conventional FACS is not suitable for detection of small sized-MVs. Indeed statistical analysis indicated that the number of CD41+MVs detected by AFM correlated only weakly with the number detected by FACS (r=0.17, P>0.05).

With AFM we found that the diameter of CD41⁺MVs isolated from fresh PPP ranges from 10-475 nm, with the majority clustering at 67.5 ± 26.5 nm. Siedlecki et al (36) also used AFM to analyse PMPs and used Nanoscope III software to calculate the size distribution of these MVs. They reported that the majority of the PMPs have a diameter of 125±21 nm and a height of 5.2±3.6 nm. They produced the PMPs by contact activation of isolated platelets on glass and on low density polyethylene, while we isolated MVs directly from PPP. This might explain the difference in MVs diameter found in both studies. Similar to their finding we observed that the height of MVs was much smaller than their diameter (z=17.4±8.6 nm), indicating that MVs bound to mica have a non-spherical shape which may be related to the process of binding to the antibody-coated mica surface. The shape of adhering (or bound) vesicles is apparently governed by the balance between bending and adhesion energies (37). Richter and Brisson (38) observed that the apparent inner height of attached vesicles corresponds to only 20% of the vesicle's original inner diameter.
As we and others have shown that about 80% of plasma MVs express platelet antigens as determined by FACS (6, 7), we characterized MVs based on their CD41 expression using a mouse anti-CD41 antibody. However, MVs from other cells may have different characteristics. Salzer et al (39) isolated microvesicles and nanovesicles from calcium ionophore treated-erythrocytes and reported that the maximum size of these vesicles is 179 nm and 81 nm, respectively. MV size may depend on the parental cell and probably also on the mechanism by which MVs are formed.
So far, there is no generally accepted protocol for the isolation of MVs from plasma (27). In this study fresh PPP obtained after double centrifugation (2,000xg for 10 min) of citrated plasma was used for the isolation of MVs to overcome artifacts of freeze-thaw procedure (22). In the present study we have compared MV preparations isolated from fresh and frozen-thawed PPP and found that there were changes in the number and the characteristics of MV. Surprisingly, after freezing and thawing a two-fold decrease in the numbers of CD41⁺MVs was found by AFM, whereas an increase in numbers of MV was found by FACS. We do not have a satisfying explanation for this observation and further investigations are needed.
Recently the question has been raised (40) whether MVs preparations might contain exosomes, secreted microvesicles formerly present inside large multivesicular endosomes (41). Interestingly, exosomes have been reported to have a diameter ranging from 30 to 90 nm (41), whereas the diameters of the CD41⁺MVs in our study ranged from 10-475 nm. Further experiments, which are beyond the scope of the present study, using exosome specific antibodies like anti-CD63 and anti-CD81 (25, 41), and density-gradient differential ultracentrifugation are needed to investigate whether part of the MVs isolated from PPP consist of exosomes.
In this study we were able to demonstrate that AFM is a suitable method to sensitively and reproducibly detect and quantify CD41⁺MVs and that AFM detects 1000-fold more CD41⁺MVs than conventional FACS.

**Table 1**

| **MVs** | **AFM** | | | | | | | | **Flowcytometry** | |
|---|---|---|---|---|---|---|---|---|---|---|
| | **Count/100** µ**m²** | | | | | **Calculation to PPP** | | | | |
| | **CD41+MVs** | | | **control (IgG1) (x10⁶ CD41⁺MPs/L PPP)** | | | | | **(x10⁶ CD41⁺MPs/L PPP)** | |
| | **Total Image** | **Mean** | **SD** | **Image** | **Mean** | **SD** | **Mean** | **SD** | **Mean** | **SD** |
| 1a | 10 | 92 | 50 | 8 | 23 | 7 | 89654 | 55894 | 63 | 15 |
| 1b | 10 | 100 | 51 | 9 | 12 | 5 | 114029 | 59195 | 82 | 34 |
| 1c | 9 | 582 | 221 | 7 | 45 | 24 | 702201 | | 162 | 20 |
| 2 | 6 | 39 | 16 | 1 | 15 | - | 316 | 20326 | 21 | 9 |
| 3 | 7 | 264 | 81 | 5 | 53 | 7 | 276582 | 95854 | - | - |
| 4 | 4 | 352 | 82 | 5 | 63 | 33 | 378043 | 64257 | 11 | 4 |
| 5 | 9 | 321 | 263 | 9 | 47 | 66 | 358629 | 257217 | 98 | 18 |
| 6 | 10 | 175 | 113 | 7 | 60 | 18 | 150440 | 123755 | 109 | 30 |
| 7 | 10 | 371 | 214 | 6 | 112 | 35 | 337899 | 234280 | 291 | 29 |

| | | | | | | | | | | |
|---|---|---|---|---|---|---|---|---|---|---|
| AFM and FACS of CD41⁺MVs isolated from fresh PPP. AFM quantification is presented as the number of MVs per 100 µm² scanned image. For both AFM and FACS measurements the number of CD41⁺MVs was calculated per L PPP after correction for IgG control-bound MPs. | | | | | | | | | | |

### Example 4: Surface Tests using Mica

### EGTA:

Freshly cleaved mica surfaces were immersed in 1 M Hepes buffer at pH 7.6 with 10 mM Calcium Dichloride for 10 minutes to deposit a layer of calcium on the surface. Dry EGTA (ethylene glycol tetraacetic acid) was then added to the buffer until saturation occurred and the EGTA began to precipitate. This solution was allowed to incubate on the mica surface for 10 minutes whereupon the mica was removed from the solution and then rinsed with the calcium enriched buffer twice. This resulted in a functionalized, EGTA covered surface suitable for the binding of antibodies. The functionalized mica surfaces were then used immediately for binding antibodies. Antibodies are now applied as described in the antibody section below.

### Glutaraldehyde:

Mica is cleaved and then immersed in absolute ethanol. APTES (3-AMINOPROPYLTRIETHOXYSILANE) is added to the ethanol solution which contained the mica until a 3% v/v mixture is obtained and then stirred rapidly for 3 minutes, resulting in the functionalized mica. The surface of the functionalized mica was then rinsed with ethanol and then with milli-Q water. The mica surface is then placed in a solution of 10% v/v gluteraldehyde in milli-Q water for 10 minutes. The mica surface is then rinsed well with mill-Q water. This functionalized surface is extremely reactive and must be used immediately for antibody fixation. Antibodies are now applied as described in the antibody section below.

### PEG:

Mica is cleaved and then immersed in absolute ethanol. APTES was added to the ethanol solution to make a concentration of 3% ATPES v/v and stirred rapidly for 3 minutes. The surface was then rinsed with ethanol and then with milli-Q water. The surface is then placed in a small container of PBS buffer at pH 7.5 and BsPEG is added to make a 100 mM solution. The mica surface is allowed to react for 20 minutes and then it is quickly rinsed with mill-Q water. Antibodies are added as described below.

### Antibody application:

Antibodies were diluted to 0.05 mg/ml with the Hepes buffer and 20 ul of the antibody solution was placed on the functionalized mica surface and allowed to incubate for 1 hour. The surfaces were then well rinsed with the Hepes buffer to remove all unfixed antibodies. Antibody-mica surfaces were stored in Hepes buffer until they were used for the microvesicle experiments. Possible Antibodies comprise: anti- CD41, anti-P-Selectin, anti- CD62e, anti-CD142, anti-CD 144, and anti-MUC1

### Attachment of microvesicles using a microfluidic chip:

Prepared coated mica surfaces were carefully kept wet for the remainder of the experiment with Hepes buffer. A microfluidic chip according to the invention was placed on a mica surface and then clamped down to prevent leaks. Using a Harvard Apparatus PicoPlus (http://www.harvardapparatus.com) syringe pump with 1ml Becton Dickinson S.A. (http://www.db.com) Syringe set at a constant flow speed of 0.01 ml/min the channels of the microfluidic chip were subsequently rinsed with 1.5 ml Hepes buffer to make sure there were no bubbles in the lines and to ensure that the channels were clean. Said Syringe was connected to inlets and outlets made of glass capillaries using Luer-Lock^{™} Adapters and One-Piece Fittings from LabSmith (http://www.labsmith.com). Said glass capillaries were connected to said microfluidic chip as has been described in WO2008072968. Approximately 1.5 ml blood plasma was allowed to flow through the channel in the microfluidic chip, thereby contacting the coated mica surface for a period of approximately 30 minutes. The channels were then rinsed with 1.5 ml Hepes buffer to remove any microvesicles not bound to the antibodies. Subsequently, the microfluidic chip was removed and the coated surface with the attached microvesicles rinsed well with Hepes buffer. The coated surfaces were stored in the Hepes buffer until they were imaged.

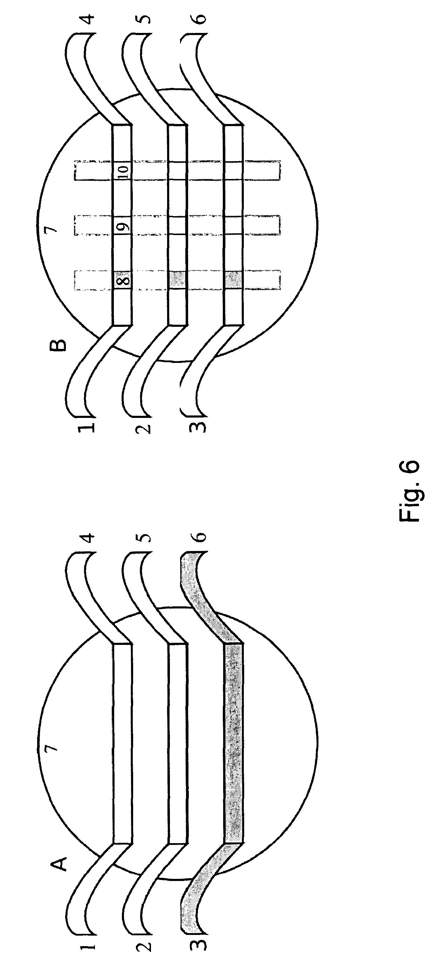

### Example 5: use of a microfluidic chip with 3 channels for the immobilization of microvesicles to a coated surface according to the invention (see figure 6).

In this method a microfluidic chip according to the invention comprising three channels applied to a mica surface (7). Subsequently, the channels are connected to a fluid comprising coating material via inlets (1), (2) and (3). Outlets (4), (5) and (6) are connected to the channels to a container, wherein the fluid is collected. Subsequently, a fluid suitable for coating a surface is flown through the channels. Subsequently, the microfluidic chip is removed from the coated surface (7) and the coated surface (7) is rotated 90 degrees and another microfluidic chip is reapplied to the coated mica surface (7) in position (6B). The 3 channels now cross the coating at positions (8), (9) and (10). Each channel is subsequently flown with a fluid comprising microvesicles. Microvesicles are now attached at positions (8), (9) and (10).

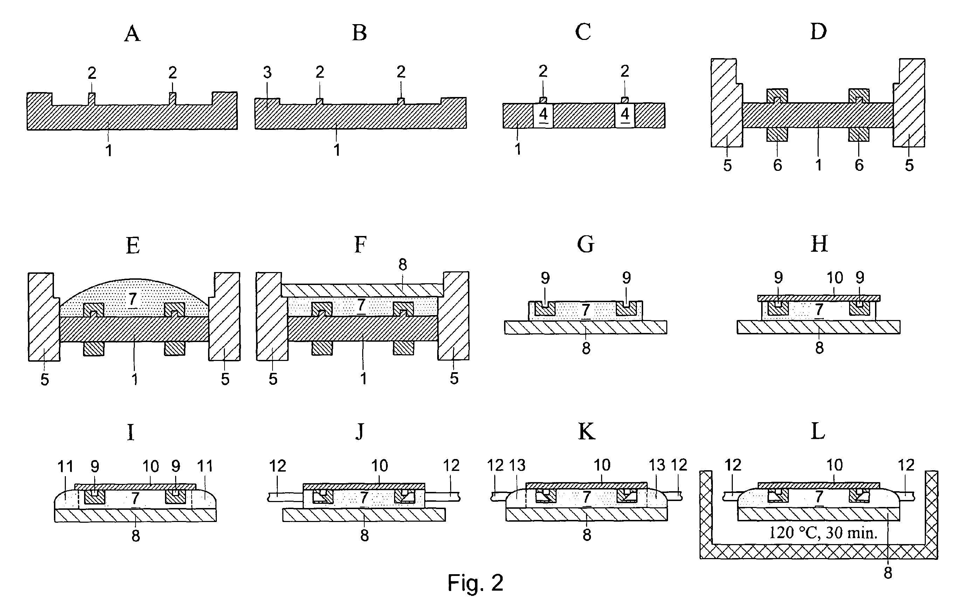

### Example 6 preparation of a chip mould and use thereof for the preparation of a microfluidic chip (see figure 2)

A mould used to create a microfluidic chip is fabricated using milling an image of a channel out of a suitable metal, preferably brass. Preferably an automated milling machine is used. First the image (1) is milled out of brass in the bottom of the mould (A). The level of the edges (2) of the bottom are kept at the same level as the top of the image in the bottom material (3) to be able to polish the surface of the top of the image (2) (figure B). Thirdly, from the bottom holes (4) are drilled, thereby completely removing the material of the bottom of the mould at these positions, resulting in 2 holes (4) (figure C). Finally little pins (6) are inserted in these holes (4) and mounted at a height of preferably half the height of the chip plus half the diameter of the glass capillary used to connect to the chip. The pins form the gaps (9) in the channels of the microfluidic chip (7) once the microchip is cast (D). Surrounding sides (5) are clamped around the bottom of the mould (1) to prevent leakage of PDMS in the following steps. Subsequently, the mould is filled with a mixture of degassed PDMS to form the cast of the microfluidic chip (7) (Dow Corning Sylgard 184, ratio 10:1), fig 2-E. Degassing of the PDMS is done by applying a vacuum to the PDMS mixture until all bubbles are gone (takes about 30 min). In order to reduce the chance of air bubbles, the filled mould is carefully covered with a glass plate (8) to create an optically flat surface (F). Curing is done at 70 degrees Celsius for one hour. The mould, consisting of elements (1-6), is removed (G) and a glass plate (10) is attached onto the cured microfluidic chip (7) to form the bottom of the microfluidic device (7 + 10) (H). Preferably, an extra layer of PDMS (11) is applied, as shown in figure 2-I. An advantage is that this extra layer provides a stronger bond between the bottom glass plate (10) and the PDMS micofluidic chip (7). To create inlets and outlets of the microfluidic chip, capillaries are connected to each microfluidic channel. To this end, capillaries made out of glass are sharpened to enable them to perforate the PDMS microfluidic chip. Capillaries are cut using a piece of aluminum oxide to create a slight scratch through a polyimide coating. Tips of the capillaries are beveled using mechanical grinding with a disc containing diamond dust. Before and after the mechanical grinding, the capillaries are rinsed with water. Before or after connecting the chip to create an inlet and an outlet, each at a different side towards the gap in the channel, the chip and capillaries are sterilized using an autoclave at 120 degrees Celsius for 30 minutes (fig 2-L). To make the connections between the capillaries and the microfluidic chip more robust, they can be secured with an extra layer of PDMS (13). An advantage is that this extra layer provides a stronger bond between the PDMS chip (7) and the glass capillaries (12).

### Reference List

1. Jimenez, J. J., W. Jy, L. M. Mauro, C. Soderland, L. L. Horstman, and Y. S. Ahn. 2003. Endothelial cells release phenotypically and quantitatively distinct microparticles in activation and apoptosis. Thromb. Res. 109: 175-180 PM:12757771.
2. Coleman, M. L., E. A. Sahai, M. Yeo, M. Bosch, A. Dewar, and M. F. Olson. 2001. Membrane blebbing during apoptosis results from caspase-mediated activation of ROCK I. Nat. Cell Biol. 3: 339-345 PM:11283606.
3. Distler, J. H., L. C. Huber, A. J. Hueber, C. F. Reich, III, S. Gay, O. Distler, and D. S. Pisetsky. 2005. The release of microparticles by apoptotic cells and their effects on macrophages. Apoptosis. 10: 731-741 PM:16133865.
4. Simak, J., K. Holada, and J. G. Vostal. 2002. Release of annexin V-binding membrane microparticles from cultured human umbilical vein endothelial cells after treatment with camptothecin. BMC. Cell Biol. 3: 11 PM:12052248.
5. Bode, A. P., and D. H. Hickerson. 2000. Characterization and quantitation by flow cytometry of membranous microparticles formed during activation of platelet suspensions with ionophore or thrombin. Platelets. 11: 259-271 PM:11030460.
6. Horstman, L. L., and Y. S. Ahn. 1999. Platelet microparticles: a wide-angle perspective. Crit Rev. Oncol. Hematol. 30: 111-142 PM:10439058.
7. Tesselaar, M. E., F. P. Romijn, d. L. van, I, F. A. Prins, R. M. Bertina, and S. Osanto. 2006. Microparticle-associated tissue factor activity: a link between cancer and thrombosis? J. Thromb. Haemost. PM:17166244.
8. VanWijk, M. J., E. VanBavel, A. Sturk, and R. Nieuwland. 2003. Microparticles in cardiovascular diseases. Cardiovasc. Res. 59: 277-287 PM:12909311.
9. Bratton, D. L., V. A. Fadok, D. A. Richter, J. M. Kailey, L. A. Guthrie, and P. M. Henson. 1997. Appearance of phosphatidylserine on apoptotic cells requires calcium-mediated nonspecific flip-flop and is enhanced by loss of the aminophospholipid translocase. J. Biol. Chem. 272: 26159-26165 PM:9334182.
10. Zwaal, R. F., P. Comfurius, and E. M. Bevers. 2005. Surface exposure of phosphatidylserine in pathological cells. Cell Mol. Life Sci. 62: 971-988 PM:15761668.
11. Hugel, B., M. C. Martinez, C. Kunzelmann, and J. M. Freyssinet. 2005. Membrane microparticles: two sides of the coin. Physiology. (Bethesda.) 20: 22-27 PM:15653836.
12. Kim, H. K., K. S. Song, J. H. Chung, K. R. Lee, and S. N. Lee. 2004. Platelet microparticles induce angiogenesis in vitro. Br. J. Haematol. 124: 376-384 PM:14717787.
13. Janowska-Wieczorek, A., M. Wysoczynski, J. Kijowski, L. Marquez-Curtis, B. Machalinski, J. Ratajczak, and M. Z. Ratajczak. 2005. Microvesicles derived from activated platelets induce metastasis and angiogenesis in lung cancer. Int. J. Cancer 113: 752-760 PM:15499615.
14. Andreola, G., L. Rivoltini, C. Castelli, V. Huber, P. Perego, P. Deho, P. Squarcina, P. Accornero, F. Lozupone, L. Lugini, A. Stringaro, A. Molinari, G. Arancia, M. Gentile, G. Parmiani, and S. Fais. 2002. Induction of lymphocyte apoptosis by tumor cell secretion of FasL-bearing microvesicles. J. Exp. Med. 195: 1303-1316 PM:12021310.
15. Kim, J. W., E. Wieckowski, D. D. Taylor, T. E. Reichert, S. Watkins, and T. L. Whiteside. 2005. Fas ligand-positive membranous vesicles isolated from sera of patients with oral cancer induce apoptosis of activated T lymphocytes. Clin. Cancer Res. 11: 1010-1020 PM:15709166.
16. Hron, G., M. Kollars, H. Weber, V. Sagaster, P. Quehenberger, S. Eichinger, P. A. Kyrle, and A. Weltermann. 2007. Tissue factor-positive microparticles: Cellular origin and association with coagulation activation in patients with colorectal cancer. Thromb. Haemost. 97: 119-123 PM:17200778.
17. Davila, M., A. Amirkhosravi, E. Coll, H. Desai, L. Robles, J. Colon, C. H. Baker, and J. L. Francis. 2008. Tissue factor-bearing microparticles derived from tumor cells: impact on coagulation activation. J. Thromb. Haemost. PM:18433463.
18. Bidot, L., W. Jy, C. Bidot, Jr., J. J. Jimenez, V. Fontana, L. L. Horstman, and Y. S. Ahn. 2008. Microparticle-mediated thrombin generation assay: increased activity in patients with recurrent thrombosis. J. Thromb. Haemost. 6: 913-919 PM:18363818.
19. Habib, A., C. Kunzelmann, W. Shamseddeen, F. Zobairi, J. M. Freyssinet, and A. Taher. 2008. Elevated levels of circulating procoagulant microparticles in patients with beta-thalassemia intermedia. Haematologica 93: 941-942 PM:18460647.
20. Aupeix, K., B. Hugel, T. Martin, P. Bischoff, H. Lill, J. L. Pasquali, and J. M. Freyssinet. 1997. The significance of shed membrane particles during programmed cell death in vitro, and in vivo, in HIV-1 infection. J. Clin. Invest 99: 1546-1554 PM:9119998.
21. Mallat, Z., B. Hugel, J. Ohan, G. Leseche, J. M. Freyssinet, and A. Tedgui. 1999. Shed membrane microparticles with procoagulant potential in human atherosclerotic plaques: a role for apoptosis in plaque thrombogenicity. Circulation 99: 348-353 PM:9918520.
22. Jy, W., L. L. Horstman, J. J. Jimenez, Y. S. Ahn, E. Biro, R. Nieuwland, A. Sturk, F. Dignat-George, F. Sabatier, L. Camoin-Jau, J. Sampol, B. Hugel, F. Zobairi, J. M. Freyssinet, S. Nomura, A. S. Shet, N. S. Key, and R. P. Hebbel. 2004. Measuring circulating cell-derived microparticles. J. Thromb. Haemost. 2: 1842-1843 PM:15456497.
23. Morel, N., O. Morel, L. Petit, B. Hugel, J. F. Cochard, J. M. Freyssinet, F. Sztark, and P. Dabadie. 2008. Generation of procoagulant microparticles in cerebrospinal fluid and peripheral blood after traumatic brain injury. J. Trauma 64: 698-704 PM:18332810.
24. Morel, O., P. Ohlmann, E. Epailly, B. Bakouboula, F. Zobairi, L. Jesel, N. Meyer, M. P. Chenard, J. M. Freyssinet, P. Bareiss, J. P. Mazzucotelli, and F. Toti. 2008. Endothelial cell activation contributes to the release of procoagulant microparticles during acute cardiac allograft rejection. J. Heart Lung Transplant. 27: 38-45 PM:18187085.
25. Heijnen, H. F., A. E. Schiel, R. Fijnheer, H. J. Geuze, and J. J. Sixma. 1999. Activated platelets release two types of membrane vesicles: microvesicles by surface shedding and exosomes derived from exocytosis of multivesicular bodies and alpha-granules. Blood 94: 3791-3799 PM:10572093.
26. Simak, J., K. Holada, A. M. Risitano, J. H. Zivny, N. S. Young, and J. G. Vostal. 2004. Elevated circulating endothelial membrane microparticles in paroxysmal nocturnal haemoglobinuria. Br. J. Haematol. 125: 804-813 PM:15180871.
27. Enjeti, A. K., L. F. Lincz, and M. Seldon. 2007. Detection and measurement of microparticles: an evolving research tool for vascular biology. Semin. Thromb. Hemost. 33: 771-779 PM:18175282.
28. Steen, H. B. 2004. Flow cytometer for measurement of the light scattering of viral and other submicroscopic particles. Cytometry A 57: 94-99 PM:14750130.
29. Furie, B., and B. C. Furie. 2006. Cancer-associated thrombosis. Blood Cells Mol. Dis. 36: 177-181 PM:16490369.
30. Parot, P., Y. F. Dufrene, P. Hinterdorfer, C. Le Grimellec, D. Navajas, J. L. Pellequer, and S. Scheuring. 2007. Past, present and future of atomic force microscopy in life sciences and medicine. J. Mol. Recognit. 20: 418-431 PM:18080995.
31. Radmacher, M., R. W. Tillmann, M. Fritz, and H. E. Gaub. 1992. From Molecules to Cells - Imaging Soft Samples with the Atomic Force Microscope. Science 257: 1900-1905 ISI:A1992JP59400022.
32. Bailey, S. W. 1984. Classification and Structures of the Micas. Reviews in Mineralogy 13: 1-12 ISI:A1984TM76400001.
33. Bailey, S. W. 1984. Crystal-Chemistry of the True Micas. Reviews in Mineralogy 13: 13-60 ISI:A1984TM76400002.
34. Muller, D. J., M. Amrein, and A. Engel. 1997. Adsorption of biological molecules to a solid support for scanning probe microscopy. J. Struct. Biol. 119: 172-188 PM:9245758.
35. Zwicker, J. I. 2008. Tissue factor-bearing microparticles and cancer. Semin. Thromb. Hemost. 34: 195-198 PM: 18645925.
36. Siedlecki, C. A., I. W. Wang, J. M. Higashi, K. Kottke-Marchant, and R. E. Marchant. 1999. Platelet-derived microparticles on synthetic surfaces observed by atomic force microscopy and fluorescence microscopy. Biomaterials 20: 1521-1529 PM:10458565.
37. Seifert, U., and R. Lipowsky. 1990. Adhesion of vesicles. Phys. Rev. A 42: 4768-4771 PM:9904586.
38. Richter, R. P., and A. R. Brisson. 2005. Following the formation of supported lipid bilayers on mica: a study combining AFM, QCM-D, and ellipsometry. Biophys. J. 88: 3422-3433 PM:15731391.
39. Salzer, U., P. Hinterdorfer, U. Hunger, C. Borken, and R. Prohaska. 2002. Ca(++)-dependent vesicle release from erythrocytes involves stomatin-specific lipid rafts, synexin (annexin VII), and sorcin. Blood 99: 2569-2577 PM:11895795.
40. Horstman, L. L., W. Jy, A. Minagar, C. J. Bidot, J. J. Jimenez, J. S. Alexander, and Y. S. Ahn. 2007. Cell-derived microparticles and exosomes in neuroinflammatory disorders. Int. Rev. Neurobiol. 79: 227-268 PM:17531844.
41. Thery, C., L. Zitvogel, and S. Amigorena. 2002. Exosomes: composition, biogenesis and function. Nat. Rev. Immunol. 2: 569-579 PM:12154376.

### Description of the figures

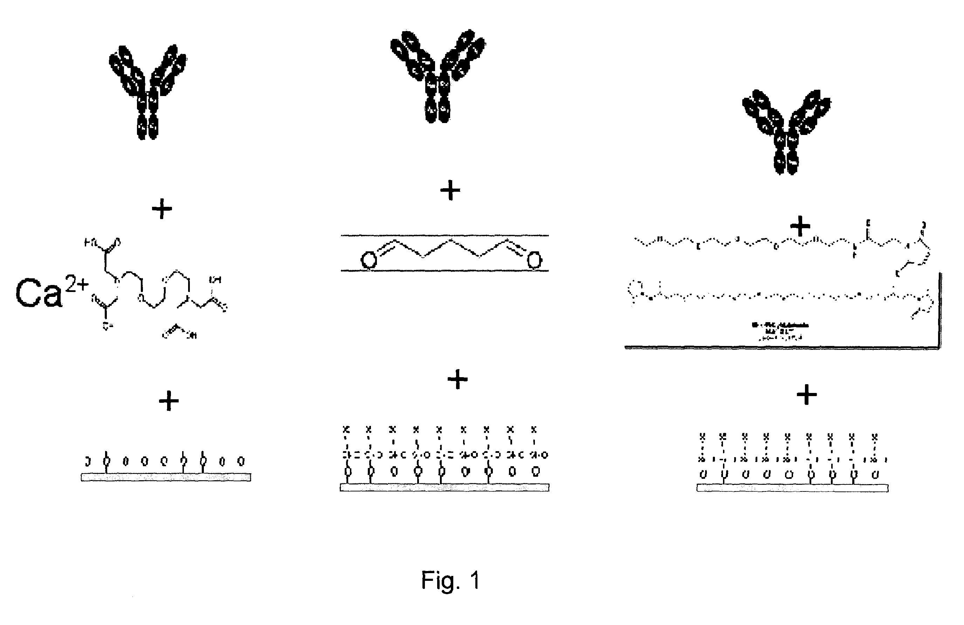

Figure 1: Surfaces tested against the efficiency of capture and prevention of plasma clotting.
Figure 2: Fabrication of the chip mould, the chip and the fluidic connection

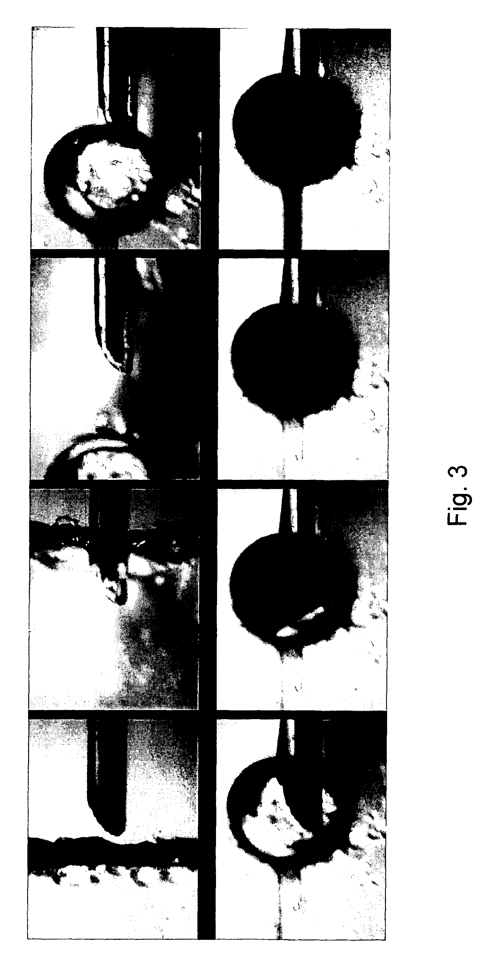

Figure 3: Movie stills of connecting a microfluidic channel

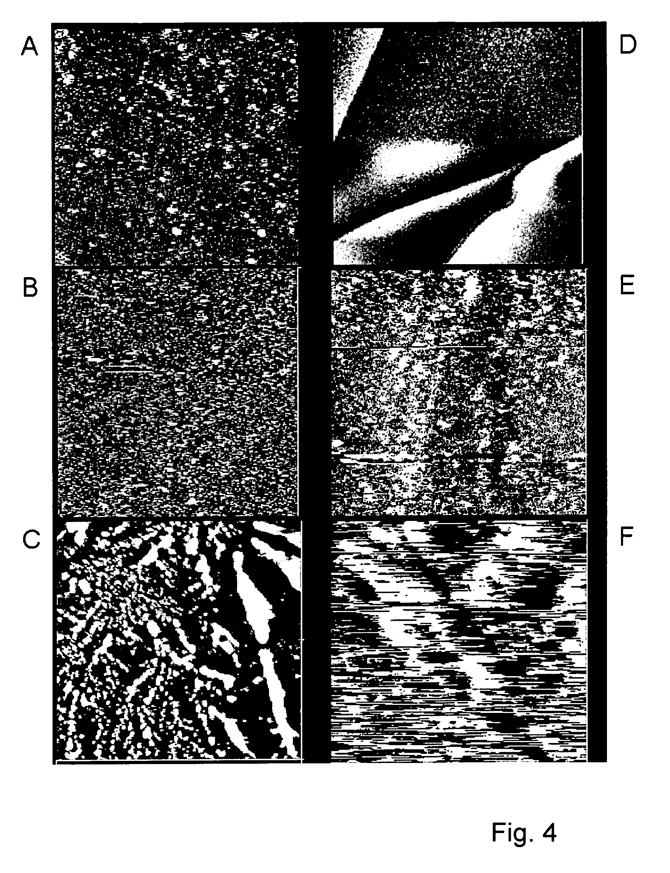

Figure 4: Left column shows the reaction of the various surfaces with anti-CD 41 and purified microparticles. On the right, we see the reaction of platelet poor plasma with IGg. A and D are EGTA treated mica. B and E show APTES and gluteraldyhyde treated surfaces. C and F show APTES and PEG coated surfaces.

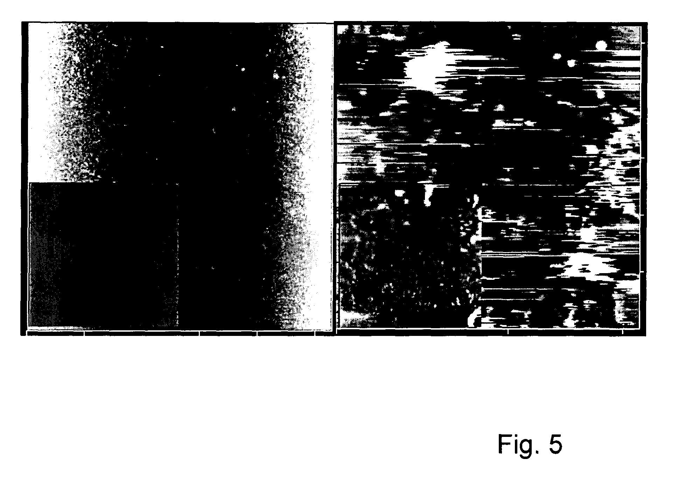

Figure 5: Left shows plasma that was dripped onto EGTA-anti-CD41 surface and allowed to sit undisturbed for 30 minutes. Right shows plasma that was flowed over the surface with microfluidics over a period of 30 minutes. Large blobs are thought to be activated platelets which are very rare in this plasma.
Figure 6: Example of the preparation of a coated surface using a microfluidic chip comprising 3 channels. In step A, a chip is positioned on a surface. The channels are used to apply a coating onto the surface. In step B the chip is repositioned and used to apply fluids comprising microvesicles onto the coated surface.

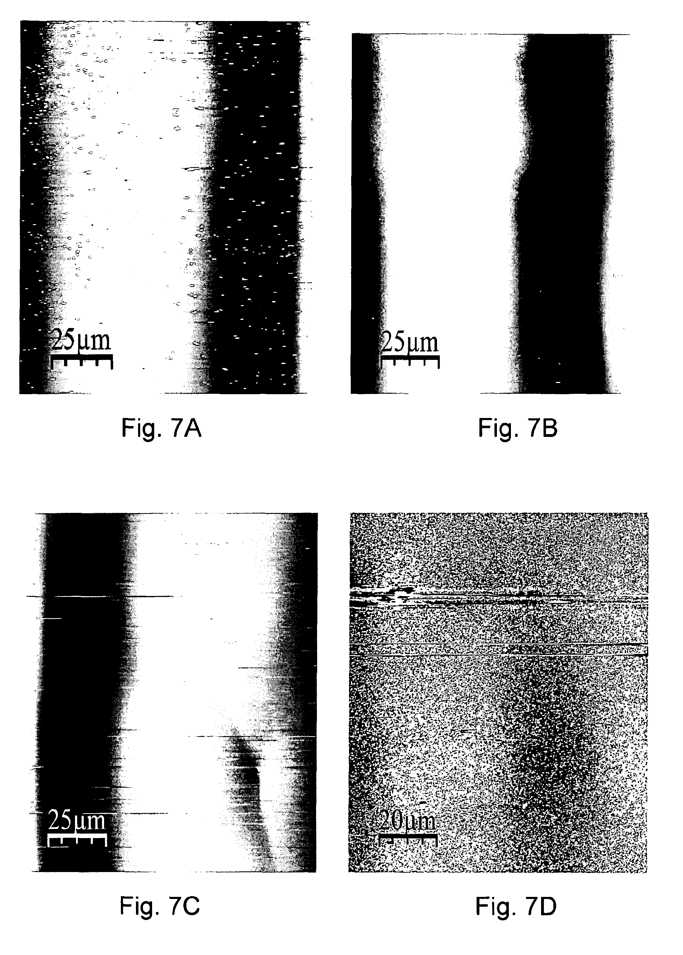

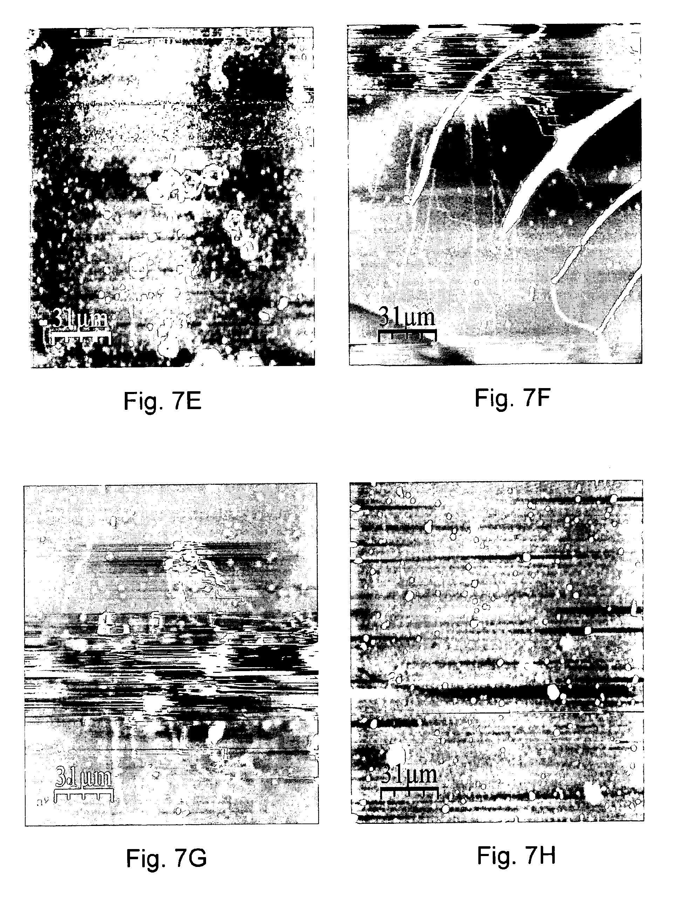

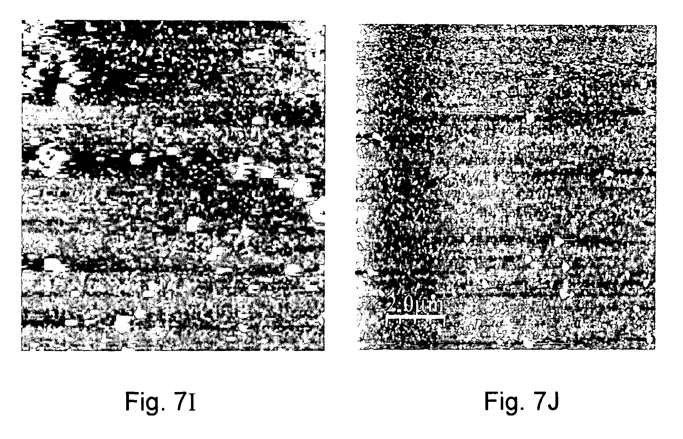

Figure 7 A-F: Example showing the effects of the microfluidics on concentrating the microvesicles by improving exposure to the surface. This figure shows the microfluidics as extraced from the platelet poor plasma (PPP) (Figure A) compared with a number of controls. Figure B is included to show the number of CD41 positive microvesicles that are typically pulled from the PPP without the concentration of the microfluidics. In figure B the PPP was applied by setting a drop of PPP 50 µl on the surface and then allowing it to incubate for one hour. The difference in the number of microvesicles that are extracted between figure A and B is clear. The plasma drop experiment shows a few particles that have been captured (small white dots) Figure B shows a smaller number of microvesicles. In figure C we see a surface before any plasma has been applied. This surface is only coated with the mouse anti-human CD41 antibody. This shows that there are no spots on this surface that would be mistaken for microvesicles. Figure D shows a surface that is prepared in the same manner as the other surfaces, only the anti-CD41 antibody is replaced with a mouse IgG1 isotype control in the same concentration and this negative control has no action against any of the objects in the plasma. The lack of microvesicles on this surface shows that the surface does not nonspecifically capture the CD41 positive microvesicles. As specificity is even more important than the raw numbers, the comparison of figure D to figure A shows that this technique is able to purify and sort only the CD41 positive microvesicles from the blood plasma. (Streaking is an artifact of the atomic force spectroscopy technique, z scales have not been normalized to facilitate comparison of the images)
   Image collection of cells and microvesicles collected from flowing the PPP through the microfluidics. The microfluidics method is more efficient than the plasma drop experiments in capturing all the different types of microvesicles in the blood. These unusual microvesicles are not seen in the plasma drop experiments, shown in figure 7 E and F, and most likely are activated platelets, the precursors of platelet microvesicles, and fibrin. The figure in the lower right side (G) shows the more common image of microvesicles that can be caught with this method. These cells are extremely rare in this plasma and show the power of this method to capture these targeted microvesicles. This shows another relevant sorting parameter available with this technique as the AFM allows sorting of the microvesicles by size, number and form.
Figure 7I shows the CD41 positive microvesicles that are collected from concentrated, isolated microvesicle preparation that has been run through the microfluidics (left). The figure on the right (J) shows the same type of microvesicle preparation dropped on the surface. In this case there are so many microvesicles in both preparations that the surface is simply saturated in a manner of minutes. It is clear that the microfluidics seem to capture larger and more varied microvesicles. The high centrifugation speed applied during microvesicle isolation helps to purify and concentrate the microvesicles from other plasma proteins, but also might change the characteristics of the microvesicles. On the other hand, the microfluidics method allows concentration and purification of the plasma in one step without any manipulation of the plasma which causes fusion, fission, and activation.
Figure 8
   The size standard polystrene beads with diameters of 0.2, 0.5, and 1 µm were measured by FACS. The light scatter patterns of these beads were plotted in one histogram and compared with the light scatter pattern of MVs.
Figure 9
   AFM image showing the three-dimensional surface topography of mica (a), modified mica (b) anti-CD41 antibody-coated mica (c) and IgG1 isotype control-coated mica (d). The inserts show the results of the height analysis (z value) of the surfaces.
Figure 10
   Three-dimensional AFM topography of CD41⁺MVs (a). The insert at the bottom represents the region denoted by the dashed box in the centre. The imaging of isotype control (IgG1)-coated mica hardly shows any MPs attached (b). The scale bar in these images is 1 µm.
Figure 11
   AFM detection of CD41⁺MVs isolated from fresh PPP showing the size distribution and amount of MVs found on a 100 µm2 scan (a). A scatter plot of the apparent height of CD41⁺MPs as a function of their corresponding diameter is presented (b).
Figure 12
   FACS plots representing the measurement of the absolute count beads (a), the detection of CD41⁺MVs (b), and IgG1-isotype control (c).

## Claims

1. A method to immobilize microvesicles ranging from 10-800 nm in diameter comprising steps of: coating a surface with molecules having an affinity for said microvesicles to obtain a coated surface; allow a fluid comprising said microvesicles to contact said coated surface by applying a laminar flow to said fluid to immobilize said microvesicles to said coated surface.

2. A method according to claim 1, wherein said affinity is specific for microvesicles.

3. A method according to any of claims 1-2, wherein said laminar flow is applied using a microfluidic chip.

4. A method according to claim 3, wherein said microfluidic chip is created using a chip mould according to anyone of claims 16-24 or produced according to a method according to claim 25.

5. A method according to any of claims 1-4, wherein said surface comprises mica.

6. A method according to any of claims 1-5, wherein said molecules having an affinity for said microvesicles comprises an antibody, preferably an anti-CD3, anti-CD4, anti-CD8, anti-CD11a, anti-CD11b, anti-CD14, anti-CD20, anti-CD31, anti-CD40, anti-CD40L, anti-CD41, anti-CD41a, anti-CD42a, anti-CD42b, anti-CD62P, anti-CD51, anti-CD54, anti-CD45, anti-CD62e, anti-CD63, anti-CD66b, anti-CD66e, anti-CD68, anti-CD81, anti-CD105 anti-CD106, anti-CD133, anti-CD142, anti-CD144, anti-CD146, anti-CD162 anti-EGFR, anti-Fas, anti-FasL, anti-glycophorin A, anti-MUC1, anti-VE Cadherin, anti-CXCR4, anti-CCR5 and/or, anti-vWF.

7. A method according to any of claims 1-6, wherein said microvesicles range in diameter between 10 and 800 nm.

8. A method according to any of claims 1-7, , wherein said microvesicles range in diameter between 10 and 200 nm.

9. A method according to any of claims 1-8, wherein the mean diameter is around 65 nm.

10. A method for the detection of microvesicles comprising steps of immobilization of microvesicles according to any of claim 1-9, said method comprising detecting said microvesicles.

11. A method according to any of claims 1-10, further comprising a step wherein at least one species of antibodies is bound to the microvesicles.

12. A method according to claim 1-11, wherein a first section of said surface is exposed to a first species of antibodies.

13. A method according to claim 12, wherein a further section of said surface is exposed to a second species of antibodies.

14. A method according to any of claims 10-13, wherein detection of microvesicles is done with an Atomic Force Microscope (AFM).

15. A method according to any of claims 10-14, wherein detection of microvesicles is done using an optical method, preferably by using a labeled antibody according to claim 6

16. A method for constructing a mould for a microfluidic chip, said method comprising providing:
- a first microfluidic chip mould comprising a bottom, and surrounding sides to form a first receptacle for fluid,
- wherein at least one side, preferably the top is at least partly open to the exterior of the first mould to allow filling of the first receptacle of the first mould,
- said bottom containing an image of a channel, a fluid inlet, a fluid outlet, and preferably one or more chambers, wherein said fluid inlet, fluid outlet and said preferably one or more chambers are in fluid connection with said channel,
- wherein said image is present on a surface of the bottom that is exposed to the fill side of said first receptacle,
- wherein said image comprises material that is in immediate contact with the surface of the bottom that is exposed to the fill side of said first receptacle,
said method further comprising
- filling said first mould with a first microfluidic chip casting material,
- curing the first casting material to create a first cast comprising a first microfluidic chip,
- separating the thus formed first microfluidic chip from the first mould, providing the first microfluidic chip with surrounding sides to form a second receptacle for fluid,
- wherein at least one side of the second receptacle, preferably the top is at least partly open to the exterior to allow filling of the second receptacle,
- wherein the surface of the first microfluidic chip containing said channel, fluid inlet, fluid outlet and preferably one or more chambers is exposed to the fill side of the second receptacle,
- filling said second receptacle with a second microfluidic chip casting material,
- curing the second casting material thereby creating a second cast comprising a second mould for said microfluidic chip, and separating said second mould from said first microfluidic chip,
- wherein said second mould comprises a copy of the image of the first mould.

17. A method according to claim 16, wherein said image comprises material that extends from said surface of the bottom that is exposed to the fill side of the first receptacle.

18. A method according to claim 16 or claim 17, wherein said first cast and said second cast are made from polymers that can be separated from each other after curing.

19. A method according to any one of claims 16-18, wherein said first cast comprises PDMS.

20. A method according to any one of claims 16-19, wherein said second cast comprises polyurethane.

21. A method according to any one of claims 16-20, comprising providing said second mould with surrounding sides to form a third receptacle for fluid,
- wherein at least one side, preferably the top of said third receptacle is at least partly open to the exterior to allow filling of the third receptacle, and
- wherein the side of said second mould that comprises said image is exposed to the fill side of said third receptacle,

22. A method according to claim 21, further comprising filling said third receptacle with a third microfluidic chip casting material and curing the third casting material to create a third cast comprising the second microfluidic chip, wherein said second cast and said third cast are made from polymers that can be separated from each other after curing.

23. A mould for a microfluidic chip cast of a deformable material, obtainable by a method according to any one of claims 16-22.

24. A mould for a microfluidic chip according to claim 23, wherein said deformable material is PDMS.

25. A method for producing a microfluidic chip, comprising filling a mould according to clams 23 or 24 with a microfluidic chip casting material, curing the microfluidic chip casting material and separating the thus formed microfluidic chip from the mould.

26. A coated surface comprising microvesicles obtainable by a method according to any of claims 1-15.

27. Use of the coated surface according to claim 26 for determining a parameter of microvesicles in a biological sample of an individual, wherein the parameter comprises the number, the dimensions and/or protein factors of said microvesicles.

28. Use according to claim 27, wherein the parameter is determined for determining whether said individual is at risk of suffering or developing a disease associated with the presence of microvesicles.[

29. Use according to claim 28, wherein said disease comprises an inflammatory disease, more preferably a systemic inflammatory disease including an autoimmune disease, diabetes mellitus or immune-mediated thrombosis, diabetes, more preferablt diabetes mellitus, a kidney disease, a cardiovascular disease, more preferably an acute coronary syndrome, diabetes mellitus, a neoplasm, more preferably cancer or a malignancy, more preferably a solid tumor or a leukemia, an infectious disease, more preferably a viral infection, a bacterial infection or a parasitic disease, a haematological disease, more preferably a sickle cell disease, a respiratory disease, a nutritional or metabolic disorder, an endocrine disease, an immunological diseases, a neurodegenerative disease or a neurological disorder. or cancer

30. Use of a microfluidic chip for the immobilization of microvesicles to a coated surface.

31. A kit suitable for use of the method according to any of claims 1-15, comprising of a microfluidic chip and a coated surface.
